(19) Europäisches Patentamt
European Patent Office
Office européen des brevets

(11) EP 1 648 896 B1

(12) **EUROPEAN PATENT SPECIFICATION**

(45) Date of publication and mention of the grant of the patent:
**13.02.2008 Bulletin 2008/07**

(21) Application number: **04741222.6**

(22) Date of filing: **22.07.2004**

(51) Int Cl.:
***C07D 487/04*** *(2006.01)* ***A61K 31/519*** *(2006.01)*
***A61P 25/22*** *(2006.01)*

(86) International application number:
**PCT/EP2004/008207**

(87) International publication number:
**WO 2005/014596 (17.02.2005 Gazette 2005/07)**

(54) **7-SUBSTITUTED 3-NITRO-PYRAZOLO [1,5-A] PYRIMIDINES**

7-SUBSTITUIERTE 3-NITRO-PYRAZOLO[1,5-A]PYRIMIDINE

3-NITRO-PYRAZOLO [1,5-A] PYRIMIDINES SUBSTITUEES EN POSITION 7

(84) Designated Contracting States:
**AT BE BG CH CY CZ DE DK EE ES FI FR GB GR HU IE IT LI LU MC NL PL PT RO SE SI SK TR**

(30) Priority: **24.07.2003 ES 200301747**
**12.07.2004 ES 200401696**

(43) Date of publication of application:
**26.04.2006 Bulletin 2006/17**

(73) Proprietor: **FERRER INTERNACIONAL, S.A.**
**08028 Barcelona (ES)**

(72) Inventors:
- **ANGLADA, Luis**
**E-08009 Barcelona (ES)**
- **PALOMER, Albert**
**E-08014 Barcelona (ES)**
- **PRINCEP, Marta**
**E-08028 Barcelona (ES)**
- **GUGLIETTA, Antonio**
**E-08750 Molins de Rei (ES)**

(74) Representative: **Reitstötter - Kinzebach**
**Patentanwälte,**
**Sternwartstrasse 4**
**81679 München (DE)**

(56) References cited:
**EP-A- 0 264 773**
**WO-A-00/59908**
**DE-A- 4 333 705**
**DE-A- 10 153 344**
**GB-A- 1 412 017**
**US-A- 5 538 977**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 648 896 B1

## Description

## Technical field

This invention is directed to agents with affinity for $GABA_A$ receptor, more specifically to pyrazolo[1,5-a] pyrimidines.

## Background of the invention

$GABA_A$ receptor (γ-aminobutyric $acid_A$) is a pentameric protein which forms a membrane ion channel. $GABA_A$ receptor is implicated in the regulation of sedation, anxiety, muscle tone, epileptogenic activity and memory functions. These actions are due to defined subunits of $GABA_A$ receptor, particularly the $\alpha_1$- and $\alpha_2$-subunits.

Sedation is modulated by the α1-subunit. Zolpidem is characterized by a high affinity for the α1-receptors and its sedative and hypnotic action is mediated by these receptors in vivo. Similarly, the hypnotic action of zaleplon is also mediated by the α1-receptors.

The anxiolytic action of diazepam is mediated by the enhancement of GABAergic transmission in a population of neurons expressing the $\alpha_2$-receptors. This indicates that the $\alpha_2$-receptors are highly specific targets for the treatment of anxiety.

Muscle relaxation in diazepam is mainly mediated by $\alpha_2$-receptors, since these receptors exhibit a highly specific expression in spinal cord.

The anticonvulsant effect of diazepam is partly due to $\alpha_1$-receptors. In diazepam, a memory-impairing compound, anterograde amnesia is mediated by $\alpha_1$-receptors.

$GABA_A$ receptor and its $\alpha_1$- and $\alpha_2$-subunits have been widely reviewed by H. Möhler et al.(J. Pharmacol. Exp. Ther., 300, 2-8, 2002); H. Möhler et al.(Curr. Opine Pharmacol., 1, 22-25, 2001); U. Rudolph et al.(Nature, 401, 796-800, 1999); and D. J. Nutt et al. (Br. J. Psychiatry, 179, 390-396, 2001).

Diazepam and other classical benzodiazepines are extensively used as anxiolytic agents, hypnotic agents, anticonvulsants and muscle relaxants. Their side effects include anterograde amnesia, decrease in motor activity and potentiation of ethanol effects.

In this context, the compounds of this invention are ligands of $\alpha_1$- and $\alpha_2$-$GABA_A$ receptor for their clinical application in sleep disorders, preferably insomnia, anxiety and epilepsy.

Insomnia is a highly prevalent disease. Its chronicity affects 10% of the population and 30% when transitory insomnia is computed as well. Insomnia describes the trouble in getting to sleep or staying asleep and is associated with hangover effects the next day such as weariness, lack of energy, low concentration and irritability. The social and health impact of this complaint is important and results in evident socioeconomic repercussions.

Pharmacological therapy in the management of insomnia firstly included barbiturates and chloral hydrate, but these drugs elicit numerous known adverse effects, for example, overdose toxicity, metabolic induction, and enhanced dependence and tolerance. In addition, they affect the architecture of sleep by decreasing above all the duration and the number of REM sleep stages. Later, benzodiazepines meant an important therapeutic advance because of their lower toxicity, but they still showed serious problems of dependence, muscle relaxation, amnesia and rebound insomnia following discontinuation of medication.

The latest known therapeutic approach has been the introduction of non-benzodiazepine hypnotics, such as pyrrolo[3,4-b]pyrazines (zopiclone), imidazo[1,2-a] pyridines (zolpidem) and, finally, pyrazolo[1,5-a] pyrimidines (zaleplon). Later, two new pyrazolo[1,5-a] pyrimidines, indiplon and ocinaplon, have entered into development, the latter with rather anxiolytic action. All these compounds show a rapid sleep induction and have less hangover effects the next day, lower potential for abuse and lower risk of rebound insomnia than benzodiazepines. The mechanism of action of these compounds is the alosteric activation of $GABA_A$ receptor through its binding to benzodiazepine binding site (C. F. P. George, The Lancet, 358, 1623-1626, 2001). While benzodiazepines are unspecific ligands at $GABA_A$ receptor binding site, zolpidem and zaleplon show a greater selectivity for $\alpha_1$-subunit. Notwithstanding that, these drugs still affect the architecture of sleep and may induce dependence in long-term treatments.

In US patent documents No. 4,626,538 and No. 6,399,621, and European Patent No. 129,847 hypnotic pyrazolo [1,5-a] pyrimidines are disclosed. These patents correspond to zaleplon, indiplon and ocinaplon, respectively.

Research for new active compounds in the management of insomnia answers an underlying health need, because even recently introduced hypnotics still affect the architecture of sleep and may induce dependence in long-term treatments.

It is therefore desirable to focus on the development of new hypnotic agents with a lower risk of side effects.

Thus, the present invention is directed to new 7-substituted 3-nitro-pyrazolo[1,5-a]pyrimidines which are active versus $GABA_A$ receptor and, particularly, versus its $\alpha_1$- and $\alpha_2$-subunits. Consequently, the compounds of this invention are useful in the treatment and prevention of all those diseases mediated by $\alpha_1$- and $\alpha_2$-$GABA_A$ receptor. Non-limitative

examples of such diseases are sleep disorders, preferably insomnia, anxiety and epilepsy. Non-limitative examples of the relevant indications of the compounds of this invention are all those diseases or conditions that need an induction of sleep, such as insomnia or anesthesia, an induction of sedation or an induction of muscle relaxation.

## Detailed description of the invention

The present invention relates to novel 7-substituted 3-nitro-pyrazolo[1,5-a]pyrimidines of general formula (I):

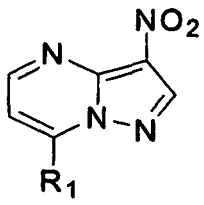

(I)

wherein

$R_1$ is selected from the group consisting of phenyl, pyridyl, pyrimidinyl, triazinyl, N-oxide-pyridyl, thienyl, furyl, thiazolyl and oxazolyl, each $R_1$ being optionally substituted with an $R_2$ group;

$R_2$ is selected from the group consisting of alkyl($C_1$-$C_6$), cycloalkyl($C_3$-$C_6$), alkenyl($C_2$-$C_6$), alkynyl($C_2$-$C_6$), alkoxy($C_1$-$C_6$), $CF_3$, CN, $SO_2$-$R_3$, $NO_2$, NH-$R_3$, $NR_3R_4$, $COR_5$, CO-$NHR_5$, $COOR_5$,

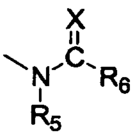 , 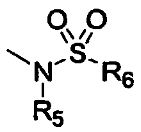 , 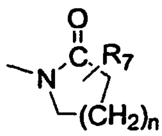 and 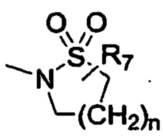 ;

$R_3$ and $R_4$ are independently selected from the group vconsisting of alkyl($C_1$-$C_6$), cycloalkyl($C_3$-$C_6$), aryl and heteroaryl;

$R_5$ is selected from the group consisting of hydrogen, alkyl($C_1$-$C_6$), alkenyl($C_2$-$C_6$), alkynyl($C_2$-$C_6$) and cycloalkyl($C_3$-$C_6$);

$R_6$ is selected from the group consisting of alkyl($C_1$-$C_6$), cycloalkyl($C_3$-$C_6$), alkoxy($C_1$-$C_6$), NH-alkyl($C_1$-$C_6$), N(dialkyl ($C_1$-$C_6$)), alkyl($C_1$-$C_6$)-O-alkyl($C_1$-$C_6$), alkyl($C_1$-$C_6$)-NH-alkyl($C_1$-$C_6$), alkyl($C_1$-$C_6$)-N(dialkyl($C_1$-$C_6$)), phenyl, monosubstituted phenyl, furyl, thienyl, thiazolyl and pyridyl;

$R_7$ is selected from the group consisting of hydrogen, alkyl($C_1$-$C_6$), cycloalkyl($C_3$-$C_6$), aryl and substituted or unsubstituted heteroaryl;

$R_8$ is selected from the group consisting of hydrogen, alkyl($C_1$-$C_6$), $CF_3$, CN, CO-$R_9$ and $SO_2$-$R_9$;

$R_9$ is selected from the group consisting of hydrogen, alkyl($C_1$-$C_6$), phenyl, substituted phenyl and substituted or unsubstituted heteroaryl;

X is O, S or $NR_8$; and

n is integer 1, 2 or 3;

and their pharmaceutically acceptable salts.

In particular, the present invention relates to novel pyrazolo[1,5-a]pyrimidines of formula (I) wherein $R_1$ is (i), (ii), (iii), (iv):

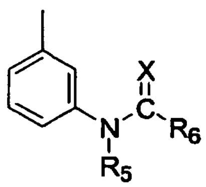

(i)

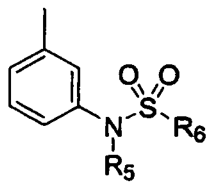

(ii)

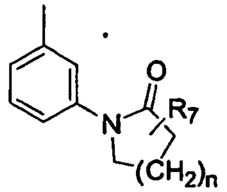
(iii)

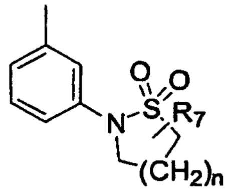
(iv) ;

phenyl, 2-trifluoromethylphenyl, 3-trifluoromethylphenyl, 4-trifluoromethylphenyl, furan-2-yl, thiophen-2-yl, pyridin-2-yl, pyridin-3-yl and pyridin-4-yl.

Preferably, in (i) and (ii) $R_5$ is selected from alkyl ($C_1$-$C_6$), cycloalkyl($C_3$-$C_6$) and alkynyl($C_2$-$C_6$) and in (iii) and (iv) $R_7$ is H and n is 1 or 2.

More particularly, in (i) and (ii) $R_5$ is selected from the group consisting of methyl, ethyl, n-propyl, i-propyl, n-butyl, cyclopropyl and 2-propynyl; and $R_6$ is selected from the group consisting of methyl, ethyl, n-propyl, i-propyl, n-butyl, phenyl and 4-methoxy-phenyl; in (iii) and (iv) $R_7$ is hydrogen and n is 1; when X is $NR_8$, $R_8$ is selected from the group consisting of hydrogen, methyl and CN.

The term "aryl" preferably includes phenyl and naphthyl. "Heteroaryl" means 5- or 6-membered aromatic heterocyclic groups containing 1, 2, or 3 heteroatoms which independently of each other are selected from N, O and S. Examples for heteroaryl groups are pyridyl, pyrimidinyl, triazinyl, pyrrolyl, imidazolyl, thiazolyl, isothiazolyl, oxazolyl, isoxazolyl, furyl, thienyl, triazolyl.

Monosubstituted phenyl means that the phenyl group carries one substituent which is selected from alkyl ($C_1$-$C_6$), alkoxy($C_1$-$C_6$), halogen, and $CF_3$.

Substituted phenyl and substituted heteroaryl means that the phenyl or heteroaryl group carries 1, 2 or 3 substituents which independently of each other are selected from alkyl($C_1$-$C_6$), alkoxy($C_1$-$C_6$), halogen, and $CF_3$. Substituted heteroaryl includes groups carrying said substituent(s) at a nitrogen heteroatom.

Halogen means fluoro, chloro, bromo, iodo and preferably fluoro and chloro.

Alkyl groups (also in alkoxy, NH-alkyl etc.) include straight chain and branched groups and preferably have 1 to 4 carbon atoms.

Preferred cycloalkyl groups are cyclopropyl, cyclopentyl and cyclohexyl.

The term "pharmaceutically acceptable salt" used herein encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids and the like.

The preferred compounds of the present invention are shown below:

N-ethyl-N-[3-(3-nitro-pyrazolo[1,5-a]pyrimidin-7-yl)-phenyl]-acetamide;
N-methyl-N-[3-(3-nitro-pyrazolo[1,5-a]pyrimidin-7-yl)-phenyl]-acetamide;
N-[3-(3-nitro-pyrazolo[1,5-a]pyrimidin-7-yl)-phenyl]-N-(n-propyl)-acetamide;
N-(n-butyl)-N-[3-(3-nitro-pyrazolo[1,5-a]pyrimidin-7-yl)-phenyl]-acetamide;
N-[3-(3-nitro-pyrazolo[1,5-a]pyrimidin-7-yl)-phenyl]-N-(2-propynyl)-acetamide;
3-nitro-7-phenyl-pyrazolo[1,5-a]pyrimidine;
3-nitro-7-(2-trifluoromethyl-phenyl)-pyrazolo[1,5-a]pyrimidine;
3-nitro-7-(3-trifluoromethyl-phenyl)-pyrazolo[1,5-a]pyrimidine;
3-nitro-7-(4-trifluoromethyl-phenyl)-pyrazolo[1,5-a] pyrimidine;
7-furan-2-yl-3-nitro-pyrazolo[1,5-a]pyrimidine;
3-nitro-7-thiophen-2-yl-pyrazolo[1,5-a]pyrimidine;
3-nitro-7-pyridin-2-yl-pyrazolo[1,5-a]pyrimidine;
3-nitro-7-pyridin-3-yl-pyrazolo[1,5-a]pyrimidine;
3-nitro-7-pyridin-4-yl-pyrazolo[1,5-a]pyrimidine;
N-ethyl-N-[3-(3-nitro-pyrazolo[1,5-a]pyrimidin-7-yl)-phenyl]-methanesulfonamide;
N-ethyl-N-[3-(3-nitro-pyrazolo[1,5-a]pyrimidin-7-yl)-phenyl]-4-methoxy-benzenesulfonamide;
N-ethyl-N-[3-(3-nitro-pyrazolo[1,5-a]pyrimidin-7-yl)-phenyl]-benzenesulfonamide;
N-methyl-N-[3-(3-nitro-pyrazolo[1,5-a]pyrimidin-7-yl)-phenyl]-methanesulfonamide;
N-(n-butyl)-N-[3-(3-nitro-pyrazolo[1,5-a]pyrimidin-7-yl)-phenyl]-4-methoxy-benzenesulfonamide;
N-[3-(3-nitro-pyrazolo[1,5-a]pyrimidin-7-yl)-phenyl]-N-(n-propyl)-4-methoxy-benzenesulfonamide;

N-methyl-N-[3-(3-nitro-pyrazolo[1,5-a]pyrimidin-7-yl)-phenyl]-4-methoxy-benzenesulfonamide;
N-(n-butyl)-N-[3-(3-nitro-pyrazolo[1,5-a]pyrimidin-7-yl)-phenyl]-4-benzenesulfonamide;
N-[3-(3-nitro-pyrazolo[1,5-a]pyrimidin-7-yl)-phenyl]-N-(n-propyl)-benzenesulfonamide;
N-methyl-N-[3-(3-nitro-pyrazolo[1,5-a]pyrimidin-7-yl)-phenyl]-4-benzenesulfonamide;
N-[3-(3-nitro-pyrazolo[1,5-a]pyrimidin-7-yl)-phenyl]-N-(n-propyl)-methanesulfonamide;
N-n-butyl)-N-[3-(3-nitro-pyrazolo[1,5-a]pyrimidin-7-yl)-phenyl]-methanesulfonamide;
1-[3-(3-nitro-pyrazolo[1,5-a]pyrimidin-7-yl)-phenyl]-pyrrolidin-2-one;
N-[3-(3-nitro-pyrazolo[1,5-a]pyrimidin-7-yl)-phenyl]-N-(prop-2-inyl)-methanesulfonamide;
N-[3-(3-nitro-pyrazolo[1,5-a]pyrimidin-7-yl)-phenyl]-N-(n-propyl)-ethanesulfonamide;
N-[3-(3-nitro-pyrazolo[1,5-a]pyrimidin-7-yl)-phenyl]-N-(n-ethyl)-ethanesulfonamide;
N-[3-(3-nitro-pyrazolo[1,5-a]pyrimidin-7-yl)-phenyl]-N-(n-prop-2-inyl)-propane-2-sulfonamide;
N-[3-(3-nitro-pyrazolo[1,5-a]pyrimidin-7-yl)-phenyl]-N-methyl-ethanesulfonamide;
N-[3-(3-nitro-pyrazolo[1,5-a]pyrimidin-7-yl)-phenyl]-N-(n-butyl)-ethanesulfonamide;
7-(3-(2-isothiazolydinyl-1,1-dioxide)-phenyl)-3-nitro-pyrazolo[1,5-a]pyrimidine;
N-[3-(3-nitro-pyrazolo[1,5-a]pyrimidin-7-yl)-phenyl]-N-methyl-propane-2-sulfonamide;
N-[3-(3-nitro-pyrazolo[1,5-a]pyrimidin-7-yl)-phenyl]-N-ethyl-propane-2-sulfonamide;
N-[3-(3-nitro-pyrazolo[1,5-a]pyrimidin-7-yl)-phenyl]-N-(n-butyl)-propane-2-sulfonamide; and
N-[3-(3-nitro-pyrazolo[1,5-a]pyrimidin-7-yl)-phenyl]-N-(n-propyl)-propane-2-sulfonamide.

Another embodiment of the present invention is to provide a process for preparing the compounds of formula (I) and their pharmaceutically acceptable salts.

Another embodiment of the present invention is the use of the compounds of formula (I) or a pharmaceutically acceptable salt thereof for preparing a pharmaceutical composition for treating or preventing diseases associated with $GABA_A$ receptor modulation.

Another embodiment of the present invention is the use of the compounds of formula (I) or a pharmaceutically acceptable salt thereof for preparing a pharmaceutical composition for treating or preventing diseases associated with $\alpha_1$-$GABA_A$ receptor modulation.

Another embodiment of the present invention is the use of the compounds of formula (I) or a pharmaceutically acceptable salt thereof for preparing a pharmaceutical composition for treating or preventing diseases associated with $\alpha_2$-$GABA_A$ receptor modulation.

Another embodiment of the present invention is the use of the compounds of formula (I) or a pharmaceutically acceptable salt thereof for preparing a pharmaceutical composition for treating or preventing anxiety.

Another embodiment of the present invention is the use of the compounds of formula (I) or a pharmaceutically acceptable salt thereof for preparing a pharmaceutical composition for treating or preventing epilepsy.

Another embodiment of the present invention is the use of the compounds of formula (I) or a pharmaceutically acceptable salt thereof for preparing a pharmaceutical composition for treating or preventing sleep disorders.

Another embodiment of the present invention is the use of the compounds of formula (I) or a pharmaceutically acceptable salt thereof for preparing a pharmaceutical composition for treating or preventing insomnia.

Another embodiment of the present invention is the use of the compounds of formula (I) or a pharmaceutically acceptable salt thereof for preparing a pharmaceutical composition for inducing sedation-hypnosis.

Another embodiment of the present invention is the use of the compounds of formula (I) or a pharmaceutically acceptable salt thereof for preparing a pharmaceutical composition for inducing anesthesia.

Another embodiment of the present invention is the use of the compounds of formula (I) or a pharmaceutically acceptable salt thereof for preparing a pharmaceutical composition for modulating the necessary time to induce sleep and its duration.

Another embodiment of the present invention is the use of the compounds of formula (I) or a pharmaceutically acceptable salt thereof for preparing a pharmaceutical composition for inducing muscle relaxation.

Another embodiment of the present invention is to provide a pharmaceutical composition containing a compound of formula (I) or a pharmaceutically acceptable salt thereof in association with therapeutically inert carriers.

The compositions include those suitable for oral, rectal and parenteral (including subcutaneous, intramuscular, and intravenous) administration, although the most suitable route will depend on the nature and severity of the condition being treated. The most preferred route of the present invention is the oral route. The compositions may be conveniently presented in unit dosage form, and prepared by any of the methods well known in the art of pharmacy.

The active compound can be combined with a pharmaceutical carrier according to conventional pharmaceutical compounding techniques. The carrier may take a wide variety of forms depending on the form of the preparation desired for administration, e.g. oral or parenteral (including intravenous injections or infusions) In preparing the compositions for oral dosage form any of the usual pharmaceutical media may be employed. Usual pharmaceutical media include, for example, water, glycols, oils, alcohols, flavoring agents, preservatives, coloring agents, and the like in the case of

oral liquid preparations (such as for example, suspensions, solutions, emulsions and elixirs); aerosols; or carriers such as starches, sugars, microcrystalline cellulose, diluents, granulating agents, lubricants, binders, disintegrating agents and the like, in the case of oral solid preparations (such as for example, powders, capsules, and tablets) with the oral solid preparations being preferred over the oral liquid preparations.

Because of their ease of administration, tablets and capsules represent the most advantageous oral dosage unit form, in which case solid pharmaceutical carriers are employed. If desired, tablets may be coated by standard aqueous or nonaqueous techniques.

A suitable dosage range for use is from about 0.01 mg to about 100,00 mg total daily dose, given as a once daily administration or in divided doses if required.

The compounds of general formula (I) may be prepared according to the reaction shown in Scheme 1.

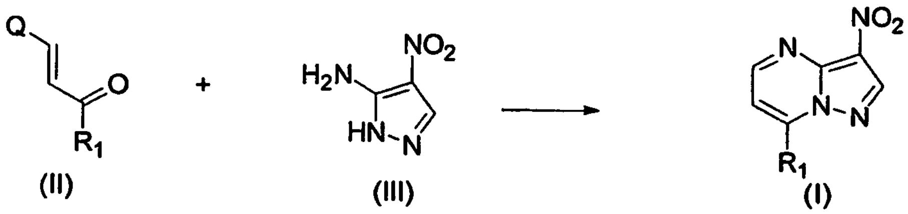

Scheme 1

where $R_1$ is as described above and Q is an appropriate leaving group consisting of dimethylamino, methylthio or methoxy. The reaction between 4-nitro-2H-pyrazol-3-ylamine (III) and appropriately substituted 1-(aryl) or (heteroaryl)-2-propen-1-one (II) is carried out in an inert polar protic or aprotic solvent such as glacial acetic acid, ethanol, methanol, dimethylformamide or dimethylsulfoxide at a temperature ranging from 50° to 130°C. After elapsing several hours (reaction time), the solvent is removed and the residue obtained is partitioned between an aqueous solution of sodium bicarbonate and dichloromethane. The crude resulting from evaporating the organic layer to dryness may be purified by one of the following methods: (a) Silica gel chromatography using ethyl acetate or dichloromethane/methanol as eluent; and (b) Crystallization in a suitable solvent (for example, ethyl acetate, ethanol, methanol, etc.).

The intermediate of formula (II) when Q is dimethylamino may be obtained by reaction between the corresponding acetophenone and N,N-dimethylformamide dimethylacetal or Bredereck's reagent (tert-butoxybis(dimethylamino)methane) as described by J. M. Domagala et al (J. Heterocyclic Chem., 26(4), 1147-58, 1989); and K. Sawada et al (Chem. Pharm. Bull., 49(7), 799-813, 2001). Specifically, when $R_1$ is a substituted aryl group, the reaction sequence leading to the intermediate of formula (II) is shown in Scheme 2, $R_5$, $R_6$, $R_7$ and n being as described above.

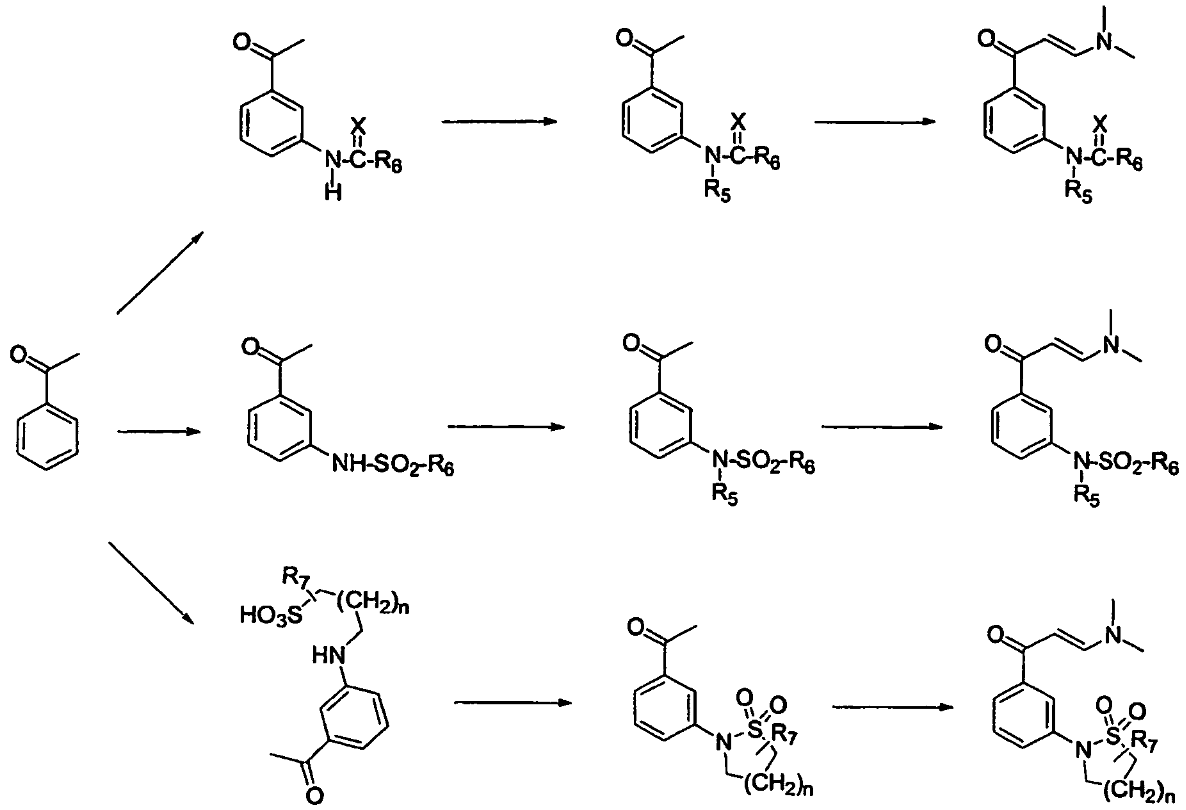

Scheme 2

The intermediate 4-nitro-2H-pyrazol-3-ylamine (III) is obtained as described by M. E. C. Biffin et al. (J. Chem. Soc. (C) 2159-2162, 1968); M. E. C. Biffin et al. (Aust. J. Chem. 26, 1041-1047, 1967); and M. E. C. Biffin et al. (Tetrahedron Lett., 21, 2029-2031, 1967) following the reaction sequences shown in Scheme 3.

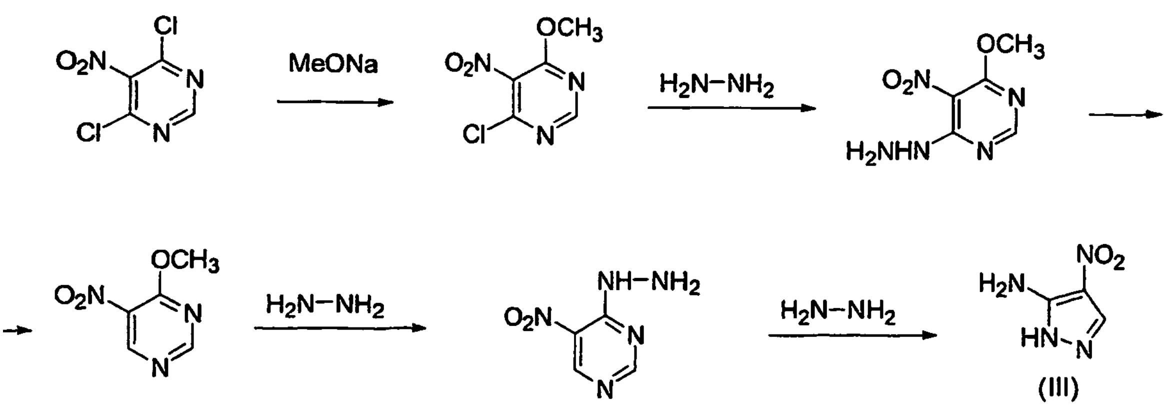


(III)

Scheme 3

From the compounds of general formula (I) it is possible to obtain their pharmaceutically acceptable salts by treatment with the corresponding acids.

The applicants have discovered that the compounds of the present invention have a high affinity for $\alpha_1$- and $\alpha_2$-GABA$_A$ receptors as shown in Tables 1 and 2. These *in vitro* results are consistent with those *in vivo* results obtained in sedation-hypnosis tests (Table 3). In accordance with the results obtained, certain compounds of the present invention have surprisingly evidenced pharmacological activity both *in vitro* and *in vivo,* which has been similar to or higher than that of prior-art compounds. All these results support!their use in diseases or conditions modulated by $\alpha_1$- and $\alpha_2$-GABA$_A$ receptors, such as insomnia or anesthesia, in which an induction of sleep, an induction of sedation or an induction of muscle relaxation are needed.
The pharmacological activity of the compounds of the present invention has been determined as shown below.

*Ligand-binding assays. Determination of the affinity of test compounds for $\alpha_1$ - and $\alpha_2$-GABA$_A$ receptors.*

Male Sprague-Dawley rats weighing 200-250 g at the time of experiment were used. After decapitation of the animal, the cerebellum (tissue that mostly contains $\alpha_1$-GABA$_A$ receptor) and spinal cord (tissue that mostly contains $\alpha_2$-GABA$_A$ receptor) were removed. The membranes were prepared according to the method by J. Lameh et al.(Prog. Neuro-Psychopharmacol. Biol. Psychiatry, 24, 979-991, 2000). Once the tissues weighed, they were suspended in 50 mM Tris HCl buffer, pH 7.7, (1:40 v/v), homogenized and then centrifuged at 20000 g for 10 min at 7°C. The resulting pellet was resuspended under the same conditions and centrifuged again. The final pellet obtained was resuspended on a minimum volume and kept at -80°C overnight. On the next day, the process was repeated until the final pellet was resuspended at a ratio of 1:10 (v/v).
The affinity of the compounds was determined by competitive tests using radiolabeled flumazenil as ligand. The methods described by S. Arbilla et al. (Eur. J. Pharmacol., 130, 257-263, 1986); and Y. Wu et al. (Eur. J. Pharmacol., 278, 125-132, 1995) were used. The membranes containing the study receptors, flumazenil (radiolabeling at a final concentration of 1 nM) and ascending concentrations of test compounds (in a total volume of 500 µl in 50 nM [ph 7.4] Tris HCl buffer) were incubated. Simultaneously, the membranes were only incubated with the radiolabeled flumazenil (total binding, 100%) and in the presence of an elevated concentration of unradiolabeled flumazenil (non-specific binding, % estimate of radiolabeled ligand). The reactions started on adding the radiolabeled ligand followed by incubation for 60 minutes at 0°C. At the end of the incubation period, the tubes were filtered using a Brandel Mod. M-48R harvester and then washed three times with cold test buffer. The harvester was fitted with a GF/B filter that retained the membranes containing the receptors and the radiolabeled ligand which had been bound to the receptors. Then the filters were removed and left till dry. Once dried, the filters were cut, placed in vials with scintillation liquid and left under stirring overnight. The next day the filters were counted using a Packard Mod. Tricarb scintillation counter.
For analysis of the results the percentage of specific binding for every concentration of test compound was calculated as follows:

$$\% \text{ specific binding} = (X-N/T-N) \times 100$$

where,
X: amount of bound ligand for every concentration of compound.
T: total binding, maximum amount bound to the radiolabeled ligand.
N: Non-specific binding, amount of radiolabeled ligand bound in a non-specific way irrespective of the receptor used.
Every concentrations of compound were tested in duplicate and their mean values were used to determine the experimental values of % specific binding versus the concentration of compound. The values thus attained were fitted to a equation for competitive assays (SigmaPlot, SPSS Inc.) and the $IC_{50}$ values (concentration of compound able to inhibit by 50% the specific binding) were calculated. Inhibition constants ($K_i$) were calculated from the $IC_{50}$ values according to Cheng-Prusoff's formula (Y. Cheng y W. H. Prusoff, Biochem. Pharmacol., 22(23), 3099-3108, 1973). Alternatively, the affinity data for subunit $\alpha_2$ are expressed as % inhibition at the concentrations of $10^{-5}$M and $10^{-7}$M. The results of these tests are given in Tables 1 and 2.

Table 1. Affinity for $\alpha_1$-GABA$_A$ receptor

| Compound | $K_i$ (nM) |
|---|---|
| Example 1 | 88.6 |
| Example 2 | 96.8 |
| Example 3 | 110.0 |
| Example 5 | 38.6 |

(continued)

| Compound | $K_i$ (nM) |
|---|---|
| Example 8 | 623.0 |
| Example 15 | 11.1 |
| Example 18 | 28.3 |
| Example 25 | 101.7 |
| Example 28 | 11.7 |
| Example 31 | 48.5 |
| Example 32 | 31.0 |
| Example 34 | 165.2 |
| Example 35 | 41.2 |
| Zaleplon | 198.9 |

Table 2. Affinity for $\alpha_2$-$GABA_A$ receptor

| Compound | $K_i$ (nM) | |
|---|---|---|
| Example 1 | 499.6 | |
| Example 2 | 711.4 | |
| Example 3 | 680.4 | |
| Example 5 | 111.8 | |
| Example 15 | 295.8 | |
| Example 18 | 988.7 | |
| Example 25 | 764.1 | |
| Zaleplon | 1302.5 | |
| Compound | % Inhibition $10^{-5}$M | % Inhibition $10^{-7}$M |
| Example 28 | 96.4 | 29.0 |
| Example 31 | 81.3 | 4.2 |
| Example 32 | 89.0 | 21.0 |
| Example 34 | 86.9 | 4.3 |
| Example 35 | 91.5 | 18.9 |
| Zaleplon | 78.4 | --- |

*In vivo determination of predictive sedative-hypnotic action.*

The *in vivo* effects of these compounds were assessed by a predictive sedation-hypnosis test in mice (D. J. Sanger et al., Eur. J. Pharmacol., 313, 35-42, 1996; and G. Griebel et al., Psychopharmacology, 146, 205-213, 1999). Groups of 5-8 male CD1 mice, weighing 22-26 g at the time of test, were used. The test compounds were administered in single equimolecular intraperitoneal doses, suspended in 0.25% agar with one drop of Tween in a volume of 10 ml/kg. Control animals received the vehicle alone. Using an Actisystem DAS16 (Panlab, S.L., Spain) the crossings (number of counts) were recorded for each mouse at 5-min intervals during a period of 30 minutes after dosing. The inhibition percentage of crossings of treated animals versus control animals (the first 5 min were discarded) was calculated. The results of this test are given in Table 3.

Table 3. Determination of sedation-hypnosis in mice.

| Compound | % Inhibition Motor Activity |
|---|---|
| Example 1 | 77.25 |
| Example 2 | 77.25 |
| Example 3 | 61.68 |
| Example 5 | 79.06 |
| Example 8 | 69.08 |
| Example 18 | 68.55 |

(continued)

| Compound | % Inhibition Motor Activity |
|---|---|
| Example 25 | 61.06 |
| Example 28 | 94.19 |
| Example 31 | 94.31 |
| Example 32 | 91.57 |
| Example 34 | 64.23 |
| Example 35 | 91.21 |
| Zaleplon | 47.17 |

The present invention is illustrated by the following examples which are not intended to be limitative thereof.

**Example 1:** N-ethyl-N-[3-(3-nitro-pyrazolo[1,5-a]pyrimidin-7-yl)-phenyl]-acetamide

A mixture of 0.52 g (4.06 mmol) of 4-nitro-2H-pyrazol-3-ylamine and 1.057 g (4.06 mmol) of N-[3-[3-(dimethylamino)-1-oxo-2-propenyl]phenyl]-N-ethyl-acetamide in 40 ml of glacial acetic acid was refluxed for 8 hours and then the solvent was removed by reduced pressure distillation. To the resulting residue were added 40 ml of dichloromethane and 20 ml of saturated sodium bicarbonate solution. The two layers were separated, and the aqueous layer was washed with 15 ml of dichloromethane. The organic layers were washed with 20 ml of water and dried over magnesium sulfate. The dichloromethane layer was evaporated to dryness to yield an oil which, in the presence of ethyl acetate, gave 225 mg of N-ethyl-N-[3-(3-nitro-pyrazolo[1,5-a]pyrimidin-7-yl)-phenyl]-acetamide as a yellow solid (yield 17%; m.p. 176-178°C).

$^1H$ NMR(400 MHz, $CDCl_3$) : δ 1.17 (3H, t, J= 6.8 Hz), 1.94 (3H, s), 3.82 (2H, q, J= 6.8 Hz), 7.31 (1H, d, J= 4.4 Hz), 7.47(1H, d, J= 7.6 Hz), 7.69 (1H, t, J= 7.6 Hz), 7.91 (1H, s), 7.96 (1H, d, J= 7.6 Hz), 8.82 (1H, s), 9.01 (1H, d, J= 4.4 Hz).
HPLC = 96.5%

**Example 2:** N-methyl-N-[3-(3-nitro-pyrazolo[1,5-a] pyrimidin-7-yl)-phenyl]-acetamide

A mixture of 0.074 g (0.58 mmol) of 4-nitro-2H-pyrazol-3-ylamine and 0.160 g (0.58 mmol) of N-[3-[3-(dimethylamino)-1-oxo-2-propenyl]phenyl]-N-methylacetamide in 15 ml of glacial acetic acid was refluxed for 8 hours and then the solvent was removed by reduced pressure distillation. To the resulting residue were added 20 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 37 mg of N-metyl-N-[3-(3-nitro-pyrazolo[1,5-a]pyrimidin-7-yl)-phenyl]-acetamide as a yellowish-white solid (yield 29%).

$^1H$ NMR(400 MHz, $CDCl_3$): δ 1.95 (3H, s), 3.35 (3H, s), 7.30 (1H, d, J= 4.8 Hz), 7.5 (1H, d J= 7.6 Hz), 7.68 (1H, t, J= 7.6 Hz), 7.93 (2H, m), 8.82 (1H, s), 9.01 (1H, d, J= 4.4 Hz).
MS (ES) *m*/*z* = 312 (MH+)
HPLC = 93%

**Example 3:** N-[3-(3-nitro-pyrazolo[1,5-a]pyrimidin-7-yl)-phenyl]-N-(n-propyl)-acetamide

A mixture of 0.051 g (0.4 mmol) of 4-nitro-2H-pyrazol-3-ylamine and 0.1 g (0.4 mmol) of N-[3-[3-(dimethylamino)-1-oxo-2-propenyl] phenyl]-N-(n-propyl)-acetamide in 5 ml of glacial acetic acid was refluxed for 8 hours and then the solvent was removed by reduced pressure distillation. To the resulting residue were added 4 ml of dichloromethane and 5 ml of saturated sodium bicarbonate solution. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 39 mg of N-[3-(3-nitro-pyrazolo[1,5-a]pyrimidin-7-yl)-phenyl]-N-(n-propyl)-acetamide as a yellow solid (yield 20%).

$^1H$ NMR(400 MHz, $CDCl_3$): δ 0.84(3H, t, J= 7.6 Hz), 1.51 (2H, m), 1.87 (3H, s), 3.65 (2H, t, J= 7.6 Hz), 7.23 (1H, d, J= 4.4 Hz), 7.39 (1H, d J= 7.6 Hz), 7.61 (1H, t, J= 7.6 Hz), 7.83 (1H, s), 7.87 (1H, d, J= 7.6 Hz), 8.87 (1H, s), 8.93 (1H, d, J= 4.4 Hz).
HPLC = 80%

**Example 4:** N-(n-butyl)-N-[3-(3-nitro-pyrazolo[1,5-a] pyrimidin-7-yl)-phenyl]-acetamide

A mixture of 0.067 g (0.52 mmol) of 4-nitro-2H-pyrazol-3-ylamine and 0.150 g (0.52 mmol) of N-(n-butyl)-N-[3-[3-(dimethylamino)-1-oxo-2-propenyl]phenyl]-acetamide in 5 ml of glacial acetic acid was refluxed for 8 hours and then the solvent was removed by reduced pressure distillation. To the resulting residue were added 4 ml of dichloromethane and 5 ml of saturated sodium bicarbonate solution. The two layers were separated, and the aqueous layer was washed with 5 ml of dichloromethane. The organic layers were washed with 5 ml of water and dried over magnesium sulfate. The dichloromethane layer was evaporated to dryness to yield an oil which was chromatographied over silica gel (eluent: dichloromethane/methanol), giving 35 mg of N-(n-butyl)-N-[3-(3-nitro-pyrazolo[1,5-a]pyrimidin-7-yl)-phenyl]-acetamide as a yellowish-white solid (yield 19%).

$^1H$ NMR(400 MHz, $CDCl_3$): δ 0.82 (3H, t, J= 7.6 Hz), 1.25 (2H, m), 1.45 (2H, m), 1.86 (3H, s), 3.68 (2H, t, J= 7.6 Hz), 7.27 (1H, d, J= 4.4 Hz), 7.4 (1H, d, J= 8 Hz), 7.62 (1H, t, J= 8 Hz), 7.85 (1H, s), 7.88 (1H, d, J= 8 Hz), 8.73 (1H, s), 8.93 (1H, d, J= 4.4 Hz).
MS (ES) *m*/*z* = 354 (MH+)
HPLC = 83%

**Example 5:** N-[3-(3-nitro-pyrazolo[1,5-a]pyrimidin-7-yl)-phenyl]-N-(2-propynyl)-acetamide

A mixture of 0.079 g (0.62 mmol) of 4-nitro-2H-pyrazol-3-ylamine and 0.168 g (0.62 mmol) of N-[3-[3-(dimethylamino)-1-oxo-2-propenyl]phenyl]-N-(2-propynyl)-acetamide in 13 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 58 mg of N-[3-(3-nitro-pyrazolo[1,5-a]pyrimidin-7-yl)-phenyl]-N-(2-propynyl)-acetamide as a yellow solid (yield 28%).

$^1H$ NMR(400 MHz, $CDCl_3$) : δ 1.98 (3H, s), 2.25 (1H, s), 2.25 (2H, s) 7.31 (1H, d, J= 4.4 Hz), 7.60 (1H, d J= 7.6 Hz), 7.71 (1H, t, J= 7.6 Hz), 8.01-8.03 (2H, m), 8.83 (1H, s), 9.01 (1H, d, J= 4.4 Hz).
MS (ES) *m*/*z* = 336 (MH+)
HPLC = 97.7%

**Example 6:** 3-nitro-7-phenyl-pyrazolo[1,5-a]pyrimidine

A mixture of 0.100 g (0.78 mmol) of 4-nitro-2H-pyrazol-3-ylamine and 0.137 g (0.78 mmol) of 3-dimethylamino-1-phenyl-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 32 mg of 3-nitro-7-phenyl-pyrazolo[1,5-a]pyrimidine as a yellowish-white solid (yield 17%).
$^1H$ NMR(400 MHz, $CDCl_3$): δ 7.62-7.65 (3H, m), 7.66 (1H, d, J= 4.8 Hz), 8.03-8.05 (2H, m), 9.05 (1H, d, J= 4.8 Hz), 9.09 (1H, s).
HPLC = 85%

**Example 7:** 3-nitro-7-(2-trifluoromethyl-phenyl)-pyrazolo [1,5-a]pyrimidine

A mixture of 0.100 g (0.78 mmol) of 4-nitro-2H-pyrazol-3-ylamine and 0.189 g (0.78 mmol) of 3-dimethylamino-1-(2-trifluoromethyl-phenyl)-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 134 mg of 3-nitro-7-(2-trifluoromethyl-phenyl)-pyrazolo[1,5-a]pyrimidine as a yellowish-white solid (yield 56%; m.p. 195-197°C).

$^1H$ NMR(400 MHz, $CDCl_3$): δ 7.19 (1H, d, J= 4.8 Hz), 7.51-7.54 (1H, m), 7.78-7.80 (1H, m), 7.91-7.94 (1H, m), 8.73 (1H, s), 9.02 (1H, d, J= 4.4 Hz).
HPLC = 89.4%

**Example 8:** 3-nitro-7-(3-trifluoromethyl-phenyl)-pyrazolo [1,5-a]pyrimidine

A mixture of 0.100 g (0.78 mmol) of 4-nitro-2H-pyrazol-3-ylamine and 0.189 g (0.78 mmol) of 3-dimethylamino-1-(3-trifluoromethyl-phenyl)-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 131 mg of 3-nitro-7-(3-trifluoromethyl-phenyl)-pyrazolo[1,5-a]pyrimidine as a yellowish-white solid (yield 54.5%; m.p. 159-161°C).

$^1$H NMR(400 MHz, $CDCl_3$) : δ 7.32 (1H, d, J= 4.8 Hz), 7.77 (1H, t, J= 7.6 Hz), 7.91 (1H, d, J= 7.6 Hz), 8.22 (1H, d, J= 7.6 Hz), 8.23 (1H, s), 8.84 (1H, s), 9.02 (1H, d, J= 4.4 Hz).
HPLC = 88.5%

**Example 9:** 3-nitro-7-(4-trifluoromethyl-phenyl)-pyrazolo [1,5-a]pyrimidine

A mixture of 0.100 g (0.78 mmol) of 4-nitro-2H-pyrazol-3-ylamine and 0.189 g (0.78 mmol) of 3-dimethylamino-1-(4-trifluoromethyl-phenyl)-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 168 mg of 3-nitro-7-(4-trifluoromethyl-phenyl)-pyrazolo[1,5-a]pyrimidine as a yellowish-white solid (yield 70%; m.p. 191-193°C).

$^1$H NMR(400 MHz, $CDCl_3$): δ 7.29 (1H, d, J= 4.8 Hz), 7.88 (2H, d, J= 8 Hz), 8.12 (2H, d, J=8 Hz), 8.84 (1H, s), 9.02 (1H, d, J= 4.4 Hz).
HPLC = 86.9%

**Example 10:** 7-furan-2-yl-3-nitro-pyrazolo[1,5-a]pyrimidine

A mixture of 0.100 g (0.78 mmol) of 4-nitro-2H-pyrazol-3-ylamine and 0.129 g (0.78 mmol) of 3-dimethylamino-1-furan-2-yl-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 152 mg of 7-furan-2-yl-3-nitro-pyrazolo[1,5-a]pyrimidine as a yellowish-white solid (yield 85%; m.p. 235-237°C).

$^1$H NMR(400 MHz, $CDCl_3$) : δ 6.79 (1H, dd, J= 4.8 and 1.6 Hz), 7.64 (1H, d, J= 4.4 Hz), 7.81 (1H, d, J= 1.2 Hz), 8.26 (1H, d, J= 3.2 Hz), 8.87 (1H, s), 8.94 (1H, d, J= 4.8 Hz).
HPLC = 93.2%

**Example 11:** 3-nitro-7-thiophen-2-yl-pyrazolo[1,5-a]pyrimidine

A mixture of 0.100 g (0.78 mmol) of 4-nitro-2H-pyrazol-3-ylamine and 0.142 g (0.78 mmol) of 3-dimethylamino-1-thiophen-2-yl-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 91 mg of 3-nitro-7-thiophen-2-yl-pyrazolo[1,5-a]pyrimidine as a yellow solid (yield 47%; m.p. 235-,237°C).

$^1$H NMR(400 MHz, $CDCl_3$): δ 7.34 (1H, dd, J= 3.6 and 1.2 Hz), 7.56 (1H, d, J= 4.8 Hz), 7.88 (1H, dd, J= 5 and 1.2 Hz), 8.41 (1H, dd, J= 4 and 1.2 Hz), 8.90 (1H, d, J= 4.8 Hz), 8.91 (1H, s).
MS (ES) *m*/*z* = 247 (MH+)
HPLC = 93.3%

**Example 12:** 3-nitro-7-pyridin-2-yl-pyrazolo[1,5-a]pyrimidine

A mixture of 0.100 g (0.78 mmol) of 4-nitro-2H-pyrazol-3-ylamine and 0.138 g (0.78 mmol) of 3-dimethylamino-1-pyridin-2-yl-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 45 mg of 3-nitro-7-pyridin-2-yl-pyrazolo[1,5-a]pyrimidine as a yellow solid (yield 24%).

$^1$H NMR(400 MHz, $CDCl_3$) : δ 7.55 (1H, dd, J= 4.8 and 2.4 Hz), 7.98 (1H, t, J= 7.6 Hz), 8.07 (1H, d, J= 4.8 Hz), 8.86 (1H, d, J= 4.8 Hz), 8.89 (1H, s), 8.95 (1H, d, J= 8 Hz), 9.06 (1H, d, J= 4 Hz).
MS (ES) *m*/*z* = 242 (MH+)
HPLC = 98.4%

**Example 13:** 3-nitro-7-pyridin-3-yl-pyrazolo[1,5-a] pyrimidine

A mixture of 0.100 g (0.78 mmol) of 4-nitro-2H-pyrazol-3-ylamine and 0.138 g (0.78 mmol) of 3-dimethylamino-1-pyridin-3-yl-propenone in 6 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 99 mg of 3-nitro-7-pyridin-3-yl-pyrazolo[1,5-a]pyrimidine as a yellow solid (yield 47%; m.p. 302-303°C).

$^1$H NMR(400 MHz, $CDCl_3$): δ 7.65-7.69 (1H, m), 7.78 (1H, d, J= 4.4 Hz), 8.45-8.48 (1H, m), 8.81 (1H, dd, J= 4.8 and 1.6 Hz), 9.01 (1H, d, J= 4.8 Hz), 9.11 (1H, s), 9.16 (1H, dd, J= 2.4 and 0.8 Hz).
HPLC = 94.1%

**Example 14:** 3-nitro-7-pyridin-4-yl-pyrazolo[1,5-a] pyrimidine

A mixture of 0.105 g (0.82 mmol) of 4-nitro-2H-pyrazol-3-ylamine and 0.144 g (0.82 mmol) of 3-dimethylamino-1-pyridin-4-yl-propenone in 8 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 68 mg of 3-nitro-7-pyridin-4-yl-pyrazolo[1,5-a]pyrimidine as a yellow solid (yield 34%; m.p. 241-244°C).

$^1$H NMR(400 MHz, $CDCl_3$): δ 7.7 (1H, d, J= 4.4 Hz), 7.98-8.00 (2H, m), 8.84-8.86 (2H, m), 9.10 (1H, d, J= 4.4 Hz), 9. 11 (1H, s).
MS (ES) *m*/*z* = 242 (MH+)
HPLC = 83.6 %

**Example 15:** N-ethyl-N-[3-(3-nitro-pyrazolo[1,5-a]pyrimidin-7-yl)-phenyl]-methanesulfonamide

A mixture of 0.0086 g (0.068 mmol) of 4-nitro-2H-pyrazol-3-ylamine and 0.02 g (0.068 mmol) of N-[3-[3-(dimethylamino)-1-oxo-2-propenyl]phenyl]-N-ethyl-methanesulfonamide in 1.5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 15 mg of N-ethyl-N-[3-(3-nitro-pyrazolo[1,5-a]pyrimidin-7-yl)-phenyl]-methanesulfonamide as a yellow solid (yield 61%).

$^1$H NMR(400 MHz, DMSO-$d_6$): δ 1.23 (3H, t, J= 6.8 Hz), 2.96 (3H, s), 3.83 (2H, q, J= 7.2 Hz), 7.31 (1H, d, J= 4.4 Hz), 7.62 (1H, d, J= 7.6 Hz), 7.67 (1H, t, J= 7.6 Hz), 7.98 (1H, d, J= 7.6 Hz), 8.05 (1H, s), 8.82 (1H, s), 9.01 (1H, d, J= 4.4 Hz).
MS (ES) *m*/*z* = 362 (MH+)
HPLC = 92.1%

**Example 16:** N-ethyl-N-[3-(3-nitro-pyrazolo[1,5-a]pyrimidin-7-yl)-phenyl]-4-methoxy-benzenesulfonamide

A mixture of 0.1 g (0.79 mmol) of 4-nitro-2H-pyrazol-3-ylamine and 0.305 g (0.068 mmol) of N-[3-[3-(dimethylamino)-1-oxo-2-propenyl]phenyl]-N-ethyl-4-methoxy-benzenesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 117 mg of N-ethyl-N-[3-(3-nitro-pyrazolo[1,5-a]pyrimidin-7-yl)-phenyl]-4-methoxy-benzenesulfonamide as a yellow solid (yield 33%; m.p. 209-211°C).

$^1H$ NMR(400 MHz, DMSO-$d_6$): δ 1.00 (3H, t, J= 7.2 Hz), 3.59 (2H, q, J= 7.2 Hz), 3.83 (1H, s), 7.10-7.13 (2H, m), 7.35(1H, d, J= 7.6 Hz), 7.54-7.56 (2H, m), 7.60 (1H, d, J= 4.4 Hz), 7.62 (1H, t, J= 8 Hz), 7.78 (1H, s), 8.00 (1H, d, J= 8 Hz), 9.05 (1H, d, J= 4.4 Hz) 9.06 (1H, s).
HPLC = 90.4%

**Example 17:** N-ethyl-N-[3-(3-nitro-pyrazolo[1,5-a]pyrimidin-7-yl)-phenyl]-benzenesulfonamide

A mixture of 0.121 g (0.958 mmol) of 4-nitro-2H-pyrazol-3-ylamine and 0.340 g (0.958 mmol) of N-[3-[3-(dimethylamino)-1-oxo-2-propenyl]phenyl]-N-ethylbenzene-sulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 150 mg of N-ethyl-N-[3-(3-nitro-pyrazolo[1,5-a]pyrimidin-7-yl)-phenyl]-benzene-sulfonarnide as a yellow solid (yield 38%; m.p. 189-191°C).

$^1H$ NMR(400 MHz, DMSO-$d_6$): δ 1.01 (3H, t, J= 7.2 Hz), 3.62 (2H, q, J= 7.2 Hz), 7.36 (1H, d, J= 7.2 Hz), 7.57 (1H, d, J= 4.8 Hz), 7.60-7.64 (5H, m), 7.71-7.73 (1H, m), 7.76 (1H, s), 8.00 (1H, d, J= 7.6 Hz), 9.04 (1H, d, J= 4.8 Hz), 9.07 (1H, s).
HPLC = 98.9%

**Example 18:** N-methyl-N-[3-(3-nitro-pyrazolo[1,5-a]pyrimidin-7-yl)-phenyl]-methanesulfonamide

A mixture of 0.076 g (0.60 mmol) of 4-nitro-2H-pyrazol-3-ylamine and 0.160 g (0.60 mmol) of N-[3-[3-(dimethylamino)-1-oxo-2-propenyl]phenyl]-N-methyl-methanesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 107 mg of N-methyl-N-[3-(3-nitro-pyrazolo[1,5-a]pyrimidin-7-yl)-phenyl]-4-methanesulfonamide as a yellow solid (yield 54%).

$^1H$ NMR(400 MHz, DMSO-$d_6$): δ 2.93 (3H, s,), 3.42 (3H, s), 7.31 (1H, d, J= 4.8 Hz), 7.64-7.65 (2H, m), 7.91-7.93 (1H, m), 8.08 (1H, s), 8.81 (1H, s), 8.99 (1H, d, J= 4.8 Hz).
MS (ES) *m*/*z* = 348 (MH+)
HPLC = 91.7%

**Example 19:** N-(n-butyl)-N-[3-(3-nitro-pyrazolo[1,5-a] pyrimidin-7-yl)-phenyl]-4-methoxy-benzenesulfonamide

A mixture of 0.049 g (0.38 mmol) of 4-nitro-2H-pyrazol-3-ylamine and 0.160 g (0.52 mmol) of N-(n-butyl)-N-[3-[3-(dimethylamino)-1-oxo-2-propenyl]phenyl]-4-methoxy-benzenesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 90 mg of N-(n-butyl)-N-[3-(3-nitro-pyrazolo[1,5-a]pyrimidin-7-yl)-phenyl]-4-methoxy-benzenesulfonamide as a yellow solid (yield 49%; m.p. 189-190°C).

$^1H$ NMR(400 MHz, DMSO-$d_6$): δ 0.82 (3H, t, J= 7.2 Hz), 1.26-1.33 (4H, m), 3.54 (2H, t, J= 6.4 Hz), 3.83 (3H, s), 7.11 (2H, d, J= 6.8 Hz), 7.35 (1H, d J= 7.2 Hz), 7.54 (2H, d, J= 6.8 Hz), 7.58 (1H, d, J= 4.8 Hz), 7.62 (1H, t, J= 8 Hz), 7.77 (1H, s), 7.99 (1H, d, J= 7.2 Hz), 9.04 (1H, d, J= 4.4 Hz), 9.05 (1H, s).

MS (ES) $m/z$ = 482 (MH+)
HPLC = 98.4%

**Example 20:** N-[3-(3-nitro-pyrazolo[1,5-a]pyrimidin-7-yl)-phenyl]-N-(n-propyl)-4-methoxy-benzenesulfonamide

A mixture of 0.067 g (0.52 mmol) of 4-nitro-2H-pyrazol-3-ylamine and 0.210 g (0.52 mmol) of N-[3-[3-(dimethylamino)-1-oxo-2-propenyl]phenyl]-N-(n-propyl)-4-methoxy-benzene-sulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 139 mg of N-[3-(3-nitro-pyrazolo[1,5-a]pyrimidin-7-yl)-phenyl]-N-(n-propyl)-4-methoxy-benzenesulfonamide as a yellow solid (yield 57%; m.p. 184-185°C).

$^1$H NMR(400 MHz, DMSO-$d_6$): δ 0.84 (3H, t, J= 7.2 Hz), 1.32-1.37 (2H, m), 3.50 (2H, t, J= 7.2 Hz), 3.83 (3H, s), 7.11 (2H, d, J= 6.8 Hz), 7.36 (1H, d J= 7.2 Hz), 7.53 (2H, d, J= 6.8 Hz), 7.58 (1H, d, J= 4.8 Hz), 7.62 (1H, t, J= 8 Hz), 7.77 (1H, s), 7.99 (1H, d, J= 7.6 Hz), 9.04 (1H, d, J= 4.8 Hz), 9.05 (1H, s).
MS (ES) $m/z$ = 468 (MH+)
HPLC = 98.9 %

**Example 21:** N-methyl-N-[3-(3-nitro-pyrazolo[1,5-a]pyrimidin-7-yl)-phenyl]-4-methoxy-benzenesulfonamide

A mixture of 0.027 g (0.21 mmol) of 4-nitro-2H-pyrazol-3-ylamine and 0.80 g (0.21 mmol) of N-methyl-N-[3-[3-(dimethylamino)-1-oxo-2-propenyl]phenyl]-9-methoxy-benzene-sulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 50 mg of N-methyl-N-[3-(3-nitro-pyrazolo[1,5-a]pyrimidin-7-yl)-phenyl]-4-methoxy-benzenesulfonamide as a yellow solid (yield 53%; m.p. 205-206°C).

$^1$H NMR(400 MHz, DMSO-$d_6$): δ 3.15 (3H, s), 3.83 (3H, s), 7.11 (2H, d, J= 6.8 Hz), 7.36 (1H, d J= 7.2 Hz), 7.49 (2H, d, J= 6.8 Hz), 7.59 (1H, d, J= 4.8 Hz), 7.60 (1H, t, J= 7.8 Hz), 7.84 (1H, s), 7.96(1H, d, J= 7.6 Hz), 9.04 (1H, d, J= 4.4 Hz), 9.07 (1H, s).
MS (ES) $m/z$ = 440 (MH+)
HPLC = 97%

**Example 22:** N-(n-butyl)-N-[3-(3-nitro-pyrazolo[1,5-a]pyrimidin-7-yl)-phenyl]-4-benzenesulfonamide

A mixture of 0.103 g (0.80 mmol) of 4-nitro-2H-pyrazol-3-ylamine and 0.31 g (0.52 mmol) of N-(n-butyl)-N-[3-[3-(dimethylamino)-1-oxo-2-propenyl]phenyl]-benzenesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 185 mg of N-(n-butyl)-N-[3-(3-nitro-pyrazolo[1,5-a]pyrimidin-7-yl)-phenyl]-benzenesulfonamide as a yellow solid (yield 51%; m.p. 159-160°C).

$^1$H NMR(400 MHz, DMSO-$d_6$): δ 0.82 (3H, t, J= 7.2 Hz), 1.26-1.33 (4H, m), 3.57 (2H, t, J= 6.4 Hz), 7.38 (1H, d J= 8 Hz), 7.55 (1H, d, J= 4.8 Hz), 7.59-7.63 (5H, m), 7.70-7.72 (1H, m), 7.75 (1H, s), 7.99 (1H, d, J= 8 Hz), 9.03 (1H, d, J= 4.8 Hz), 9.05 (1H, s).
MS (ES) $m/z$ = 452 (MH+)
HPLC = 100%

**Example 23:** N-[3-(3-nitro-pyrazolo[1,5-a]pyrimidin-7-yl)-phenyl]-N-(n-propyl)-benzenesulfonamide

A mixture of 0.117 g (0.91 mmol) of 4-nitro-2H-pyrazol-3-ylamine and 0.340 g (0.91 mmol) of N-[3-[3-(dimethylamino)-1-oxo-2-propenyl]phenyl]-N-(n-propyl)-benzenesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over

magnesium sulfate. The dichloromethane layer was evaporated to dryness to yield an oil which, in the presence of ethyl acetate, gave 154 mg of N-[3-(3-nitro-pyrazolo[1,5-a]pyrimidin-7-yl)-phenyl]-N-(n-propyl)-benzenesulfonamide as a yellow solid (yield 39%; m.p. 154-156°C).

$^1$H NMR(400 MHz, DMSO-$d_6$) : δ 0.84 (3H, t, J= 7.2 Hz), 1.3-1.39 (2H, m), 3.53 (2H, t, J= 6.8 Hz), 7.38 (1H, d J= 8 Hz), 7.56 (1H, d, J= 4.8 Hz), 7.60-7.64 (5H, m), 7.71-7.74 (1H, m), 7.75 (1H, s), 8.00 (1H, d, J= 8.4 Hz), 9.04 (1H, d, J= 4.8 Hz), 9.06 (1H, s).
MS (ES) *m/z* = 438 (MH+)
HPLC = 100%

**Example 24:** N-methyl-N-[3-(3-nitro-pyrazolo[1,5-a]pyrimidin-7-yl)-phenyl]-4-benzenesulfonamide

A mixture of 0.78 g (0.61 mmol) of 4-nitro-2H-pyrazol-3-ylamine and 0.21 g (0.52 mmol) of N-methyl-N-[3-[3-(dimethylamino)-1-oxo-2-propenyl]phenyl]-benzenesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 108 mg of N-methyl-N-[3-(3-nitro-pyrazolo [1,5-a]pyrimidin-7-yl)-phenyl]-benzenesulfonamide as a yellow solid (yield 43%; m.p. 177-179°C).

$^1$H NMR(400 MHz, DMSO-$d_6$): δ 3.19 (3H, s), 7.39 (1H, d, J= 8 Hz), 7.57-7.63 (6H, m), 7.71 (1H, t, J= 6.8 Hz), 7.82 (1H, s), 7.95 (1H, d, J= 8 Hz), 9.04 (1H, d, J= 4.8 Hz), 9.07 (1H, s).
MS (ES) *m/z* = 409 (MH+)
HPLC = 98.2%

**Example 25:** N-[3-(3-nitro-pyrazolo[1,5-a]pyrimidin-7-yl)-phenyl]-N-(n-propyl)-methanesulfonamide

A mixture of 0.078 g (0.61 mmol) of 4-nitro-2H-pyrazol-3-ylamine and 0.19 g (0.61 mmol) of N-[3-[3-(dimethylamino)-1-oxo-2-propenyl]phenyl]-N-(n-propyl)-methanesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 118 mg of N-[3-(3-nitro-pyrazolo[1,5-a]pyrimidin-7-yl)-phenyl]-N-(n-propyl)-methanesulfonamide as a yellow solid (yield 53%; m.p. 165-167°C).

$^1$H NMR(400 MHz, DMSO-$d_6$): δ 0.90 (3H, t, J= 7.2 Hz), 1.42-1.47 (2H, m), 3.07 (3H, s), 3.68 (2H, t, J= 7.2 Hz), 7.67-7.72 (2H, m), 7.75 (1H, d, J= 4.4 Hz), 8.05-8.08 (1H, m), 8.09 (1H, s), 9.10 (1H, d, J= 4.4 Hz), 9.14 (1H, s).
MS (ES) *m/z* = 376 (MH+)
HPLC = 98.3%

**Example 26:** N-(n-butyl)-N-[3-(3-nitro-pyrazolo[1,5-a]pyrimidin-7-yl)-phenyl]-methanesulfonamide

A mixture of 0.079 g (0.61 mmol) of 4-nitro-2H-pyrazol-3-ylamine and 0.20 g (0.61 mmol) of N-(n-butyl)-N-[3-[3-(dimethylamino)-1-oxo-2-propenyl]phenyl]-methanesulfonamide in 5 ml of glacial acetic acid was refluxed for 8 hours and then the solvent was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which, in the presence of ethyl acetate, gave 135 mg of N-(n-butyl)-N-[3-(3-nitro-pyrazolo[1,5-a]pyrimidin-7-yl)-phenyl]-methanesulfonamide as a yellow solid (yield 56%; m.p. 153-155°C).

$^1$H NMR(400 MHz, DMSO-$d_6$): δ 0.84 (3H, t, J= 6.8 Hz), 1.28-1.39 (4H, m), 3.03 (3H, s), 3.68 (2H, t, J= 6.8 Hz), 7.63-7.69 (2H, m), 7.71 (1H, d, J= 4.8 Hz), 8.01-8.06 (1H, m), 8.07 (1H, s), 9.07 (1H, d, J= 4.4 Hz), 9.09 (1H, s).
MS (ES) *m/z* = 390 (MH+)
HPLC = 95.1%

**Example 27:** 1-[3-(3-nitro-pyrazolo[1,5-a]pyrimidin-7-yl)-phenyl]-pyrrolidin-2-one

A mixture of 0.100 g (0.78 mmol) of 4-nitro-2H-pyrazol-3-ylamine and 0.202 g (0.78 mmol) of 1-[3-(3-dimethylamino-acryloyl)-phenyl]-pyrrolidin-2-one in 8 ml of glacial acetic acid was refluxed for 8 hours and then the solvent

was removed by reduced pressure distillation. To the resulting residue were added 10 ml of dichloromethane and 10 ml of saturated sodium bicarbonate solution. The two layers were separated, and the aqueous layer was washed with 10 ml of dichloromethane. The organic layers were washed with 10 ml of water and dried over magnesium sulfate. The dichloromethane layer was evaporated to dryness to yield an oil which was chromatographied over silica gel (eluent: dichloromethane/methanol), giving 73 mg of 1-[3-(3-nitro-pyrazolo[1,5-a]pyrimidin-7-yl)-phenyl]-pyrrolidin-2-one as a yellow solid (yield 29%; m.p. 226-228°C).

$^1$H NMR(400 MHz, $CDCl_3$): δ 2.21-2.25 (2H, m), 2.66 (2H, t, J= 8 Hz), 3.94 (2H, t, J= 7.2 Hz), 7.30 (1H, d, J= 4.4 Hz), 7.6 (1H, t, J= 8 Hz), 7.72-7.77 (2H, m), 8.47-8.48 (1H, m), 8.82 (1H, s), 8. 97 (1H, d, J= 4.4 Hz).
MS (ES) *m*/*z* = 324 (MH+)
HPLC = 100%

**Example 28:** N-[3-(3-nitro-pyrazolo[1,5-a]pyrimidin-7-yl)-phenyl]-N-(prop-2-inyl)-methanesulfonamide

0.042 g (0.33 mmol) of 4-nitro-2H-pyrazol-3-ylamine and 0.1 g (0.33 mmol) of N-[3-[3-(dimethylamino)-1-oxo-2-propenyl] phenyl]-N-(n-prop-2-inyl)-methane-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 62 mg of N-[3-(3-nitro-pyrazolo[1,5-a]pyrimidin-7-yl)-phenyl]-N-(prop-2-inyl)-methane-sulfonamide as a yellow solid (yield 51%).

$^1$H NMR(400 MHz, $CDCl_3$): δ 2.55 (1H, t, J= 2.4 Hz), 3.11 (3H, s), 4.54 (2H, s), 7.31 (1H, d, J= 4.8 Hz), 7.67 (1H, t, J= 8 Hz), 7.89-7.92 (1H, m), 7.99-8.02 (1H, m), 8.26-8.28 (1H, m), 8.83 (1H, s), 9 (1H, d, J= 4.4 Hz).
MS (ES) *m*/*z* = 372 (MH+)
HPLC = 88.5%

**Example 29:** N-[3-(3-nitro-pyrazolo[1,5-a]pyrimidin-7-yl)-phenyl]-N-(n-propyl)-ethanesulfonamide

0.028 g (0.26 mmol) of 4-nitro-2H-pyrazol-3-ylamine and 0.07 g (0.26 mmol) of N-[3-[3-(dimethylamino)-1-oxo-2-propenyl]phenyl]-N-(n-propyl)-ethanesulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 24 mg of N-[3-(3-nitro-pyrazolo[1,5-a] pyrimidin-7-yl)-phenyl]-N-(propyl)-ethanesulfonamide as a yellow solid (yield 29%).

$^1$H NMR(400 MHz, $CDCl_3$): δ 0.94 (3H, t, J= 7.6 Hz), 1.42 (3H, T, J= 7.6 Hz), 1.54-1.60 (2h, m), 3.06-3.12 (2H, q, J= 7.6 Hz), 3.74 (2H, T, J= 7.6 Hz), 7.31 (1H, d, J= 4.4 Hz), 7.61-7.67 (2H, m), 7.95-7.98 (1H, m), 8.06-8.07 (1H, m), 8.83 (1H, s), 8.98-8.99 (1H, d, J= 4.4 Hz).
MS (ES) m/z = 390 (MH+)
HPLC = 96.1%

**Example 30:** N-[3-(3-nitro-pyrazolo[1,5-a]pyrimidin-7-yl)-phenyl]-N-(ethyl)-ethanesulfonamide

0.029 g (0.23 mmol) of 4-nitro-2H-pyrazol-3-ylamine and 0.07 g (0.23 mmol) of N-[3-[3-(dimethylamino)-1-oxo-2-propenyl]phenyl]-N-(ethyl)-ethanesulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 21 mg of N-[3-(3-nitro-pyrazolo[1,5-a] pyrimidin-7-yl)-phenyl]-N-(n-ethyl)-ethanesulfonamide as a yellow solid (yield 25%)

$^1$H NMR (400 MHz, $CDCl_3$) : δ 1.22 (3H, t, J= 7.2 Hz), 1.4 (6H, d, J= 7.2 Hz), 3.28 (1H, m), 3.86 (2H, t, J= 6.8 Hz), 7.31 (1H, d, J= 4.4 Hz), 7.63-7.65 (2H, m), 7.94-7.97 (1H, m), 8.06-8.08 (1H, m), 8.82 (1H, s), 8.98-8.99 (1H, d, J= 4.4 Hz).
MS (ES) m/z = 376 (MH+)
HPLC = 96.4%

**Example 31:** N-[3-(3-nitro-pyrazolo[1,5-a]pyrimidin-7-yl)-phenyl]-N-(n-prop-2-inyl)-propane-2-sulfonamide

0.048 g (0.37 mmol) of 4-nitro-2H-pyrazol-3-ylamine and 0.125 g (0.37 mmol) of N-[3-[3-(dimethylamino)-1-oxo-2-propenyl]phenyl]-N-(n-prop-2-inyl)-propane-2-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 37 mg of N-[3-(3-nitro-pyrazolo[1,5-a] pyrimidin-7-yl)-phenyl]-N-(n-prop-2-inyl)-propane-2-sulfonamide as a yellow solid (yield 25%).

$^1$H NMR (400 MHz, $CDCl_3$): δ 1.43 (6H, d, J= 6.4 Hz), 2.43 (1H, s), 3.44-3.5 (1H, m), 4.55 (2H, s), 7.31 (1H, d, J= 4.8 Hz), 7.65 (1H, t, J= 7.6 Hz), 7.80-7.82 (1H, m), 7.99 (1H, d, J= 7.6 Hz), 8.21 (1H, s),8.83(1H, s), 8.99 (1H, d, J= 4.4 Hz).
MS (ES) m/z = 400 (MH+)
HPLC = 100%

**Example 32:** N-[3-(3-nitro-pyrazolo[1,5-a]pyrimidin-7-yl)-phenyl]-N-methyl-ethanesulfonamide

0.043 g (0.34 mmol) of 4-nitro-2H-pyrazol-3-ylamine and 0.1 g (0.34 mmol) of N-[3-[3-(dimethylamino)-1-oxo-2-propenyl] phenyl]-N-methyl-ethanesulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 38 mg of N-[3-(3-nitro-pyrazolo[1,5-a]pyrimidin-7-yl)-phenyl]-N-(methyl)-ethanesulfonamide as a yellow solid (yield 31%).

$^1$H NMR (400 MHz, $CDCl_3$): δ 1.41 (3H, t, J= 7.2 Hz), 3.11 (2H, q, J= 7.6 Hz), 3.44 (3H, s), 7.3 (1H, d, J= 4.4 Hz), 7.59-7.67 (2H, m), 7.88-7.92 (1H, m), 8.08-8.09 (1H, m), 8.83 (1H, s), 8.99 (1H, d, J= 4.8 Hz).
MS (ES) m/z = 362 (MH+)
HPLC = 96.1%

**Example 33:** N-[3-(3-nitro-pyrazolo[1,5-a]pyrimidin-7-yl)-phenyl]-N-(n-butyl)-ethanesulfonamide

0.026 g (0.21 mmol) of 4-nitro-2H-pyrazol-3-ylamine and 0.07 g (0.21 mmol) of N-[3-[3-(dimethylamino)-1-oxo-2-propenyl]phenyl]-N-(n-butyl)-ethanesulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 34 mg of N-[3-(3-nitro-pyrazolo[1,5-a] pyrimidin-7-yl)-phenyl]-N-(n-butyl)-ethanesulfonamide as a yellow solid (yield 41%).

$^1$H NMR (400 MHz, $CDCl_3$): δ 0.91 (3H, t, J= 7.2 Hz), 1.34-1.43 (5H, m), 1.49-1.52 (2H, m), 3.09 (2H, q, J= 7.2 Hz), 3.78 (2H, t, J= 7.2 Hz), 7.31 (1H, d, J= 4.4 Hz), 7.61-7.67 (2H, m), 7.95-7.98 (1H, m), 8.06 (1H, s),8.23 (1H, s), 8.99 (1H, d, J= 4.4 Hz).
MS (ES) m/z = 404 (MH+)
HPLC = 99.1%

**Example 34:** 7-(3-(2-isothiazolidinyl-1,1-dioxide)-phenyl)-3-nitro-pyrazolo[1,5-a]pyrimidine

0.043 g (0.34 mmol) of 4-nitro-2H-pyrazol-3-ylamine and 0.1 g (0.34 mmol) of 3-dimethylamino-1-[3-(1,1-dioxoisothiazolydin-2-yl)-phenyl]-propenone dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 64 mg of 7-[3-(2-isothiazolydinyl-1,1-dioxide)-phenyl)-3-nitro-pyrazolo[1,5-a]pyrimidine as a yellow solid (yield 52%).

$^1$H NMR (400 MHz, DMSO-$d_6$) : δ 2.47-2.51 (2H, m), 3.61 (2H, t, J= 7.2 Hz), 3.86 (2H, t, J= 6.4 Hz), 7.55 (1H, d, J= 7.6 Hz), 7.67 (1H, t, J= 8 Hz), 7.7 (1H, d, J= 4.4 Hz), 7.78-7.81 (2H, m), 9.1 (1H, d, J= 4 Hz), 9.14 (1H, s).
MS (ES) m/z = 360 (MH+)
HPLC = 86.9%

**Example 35:** N-[3-(3-nitro-pyrazolo[1,5-a]pyrimidin-7-yl)-phenyl]-N-methyl-propane-2-sulfonamide

0.062 g (0.48 mmol) of 4-nitro-2H-pyrazol-3-ylamine and 0.15 g (0.48 mmol) of N-[3-[3-(dimethylamino)-1-oxo-2-propenyl]phenyl]-N-methyl-propane-2-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 122 mg of N-[3-(3-nitro-pyrazolo[1,5-a] pyrimidin-7-yl)-phenyl]-N-methyl-propane-2-sulfonamide as a yellow solid (yield 67%).

$^1H$ NMR (400 MHz, $CDCl_3$): δ 1.39 (6H, d, J= 7.2 Hz), 3.36-3.341 (1H, m), 3.46 (3H, s), 7.3 (1H, d, J= 4.4 Hz), 7.59-7.67 (2H, m), 7.85-7.88 (1H, m), 8.10-8.12 (1H, m), 8.82 (1H, s), 8.97-8.99 (1H, d, J= 4.4 Hz).
MS (ES) m/z = 376 (MH+)
HPLC = 91.6%

**Example 36:** N-[3-(3-nitro-pyrazolo[1,5-a]pyrimidin-7-yl)-phenyl]-N-ethyl-propane-2-sulfonamide

0.067 g (0.52 mmol) de 4-nitro-2H-pyrazol-3-ylamine and 0.17 g (0.52 mmol) of N-[3-[3-(dimethylamino)-1-oxo-2-propenyl]phenyl]-N-ethyl-propane-2-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 97 mg of N-[3-(3-nitro-pyrazolo[1,5-a]pyrimidin-7-yl)-phenyl]-N-ethyl-propane-2-sulfonamide as a yellow solid (yield 47%).

$^1H$ NMR (400 MHz, $CDCl_3$): δ 1.22 (3H, t, J= 7.2 Hz), 1.4 (6H, d, J= 7.2 Hz), 3.28 (1H, m), 3.86 (2H, t, J= 6.8 Hz), 7.31 (1H, d, J= 4.4 Hz), 7.63-7.65 (2H, m), 7.94-7.97 (1H, m), 8.06-8.08 (1H, m), 8.82 (1H, s), 8.98-8.99 (1H, d, J= 4.4 Hz).
MS (ES) m/z = 390 (MH+)
HPLC = 93.9%

**Example 37:** N-[3-(3-nitro-pyrazolo[1,5-a]pyrimidin-7-yl)-phenyl]-N-(n-butyl)-propane-2-sulfonamide

0.032 g (0.26 mmol) of 4-nitro-2H-pyrazol-3-ylamine and 0.09 g (0.26 mmol) of N-[3-[3-(dimethylamino)-1-oxo-2-propenyl]phenyl]-N-(n-butyl)-propane-2-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 49 mg of N-[3-(3-nitro-pyrazolo[1,5-a]pyrimidin-7-yl)-phenyl]-N-(n-butyl)-propane-2-sulfonamide as a yellow solid (yield 46%).

$^1H$ NMR(400 MHz, $CDCl_3$): δ 0.89(3H, t, J= 7.6 Hz), 1.36 (2H, m), 1.40 (2H, d, J= 6.8 Hz), 1.51 (2H, m), 3.27 (1H, m), 3.80 (2H, t, J= 7.6 Hz), 7.31 (1H, d, J= 4.4 Hz), 7.63-7.65 (2H, m), 7.94-7.96 (1H, m), 8.09(1H, m), 8.82 (1H, s), 8.89 (1H, d, J= 4.4 Hz).
MS (ES) *m*/*z* = 418 (MH+)
HPLC = 100%

**Example 38:** N-[3-(3-nitro-pyrazolo[1,5-a]pyrimidin-7-yl)-phenyl]-N-(n-propyl)-propane-2-sulfonamide

0.064 g (0.50 mmol) of 4-nitro-2H-pyrazol-3-ylamine and 0.17 g (0.50 mmol) of N-[3-[3-(dimethylamino)-1-oxo-2-propenyl]phenyl]-N-(n-propyl)-propane-2-sulfonamide dissolved in 5 ml of glacial acetic acid were refluxed for 8 hours and then the solvent was removed by reduced pressure distillation. To the resulting residue were added 4 ml of dichloromethane and 5 ml of saturated sodium bicarbonate. The two layers were separated, and the aqueous layer was washed with 5 ml of dichloromethane. The organic layers were washed with 5 ml of water and dried over magnesium sulfate. The dichloromethane layer was evaporated to dryness to yield an oil which, in the presence of ethyl acetate, gave 116 mg of N-[3-(3-nitra-pyrazala[1,5-a]pyrimidin-7-yl)-phenyl]-N-(n-propyl)-propane-2-sulfonamide as a yellow solid (yield 57%).

$^1H$ NMR (400 MHz, $CDCl_3$): δ 0.93 (3H, t, J= 7.6 Hz), 1.4 (6H, d, J= 7.2 Hz), 1.53-1.58 (2H, m), 3.26-3.29 (1H,

m), 3.76 (2H, t, J= 7.6 Hz), 7.31 (1H, d, J= 4.8 Hz), 7.63-7.65 (2H, m), 7.94-7.96 (1H, m), 8.08-8.09 (1H, m), 8.82 (1H, s).
MS (ES) m/z = 404 (MH+)
HPLC = 94.5%

**Example 39:** 5 mg tablets

| Compound of Example 1 | 5.0 mg |
|---|---|
| Colloidal silicon dioxide | 0.6 mg |
| Croscarmellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

**Example 40:** 10 mg capsules

| Compound of Example 1 | 10.0 mg |
|---|---|
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |
| Microcrystalline cellulose q.s. to | 155.0 mg |

**Example 41:** oral drops

| Compound of Example 1 | 0.5 g |
|---|---|
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified water q.s. to | 100.0 mL |

**Example 42:** 2.5 mg tablets

| Compound of Example 28 | 2.5 mg |
|---|---|
| Colloidal silicon dioxide | 0.6 mg |
| Croscarmellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

**Example 43:** 5 mg capsules

| Compound of Example 28 | 5.0 mg |
|---|---|
| Colloidal silicon dioxide | 0.6 mg |
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |
| Microcrystalline cellulose q.s. to | 155.0 mg |

**Example 44:** oral drops

| Compound of Example 28 | 0.25 g |
|---|---|
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified water q.s. to | 100.0 mL |

## Claims

**1.** A compound of formula (I):

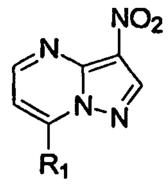

(I)

wherein

$R_1$ is selected from the group consisting of phenyl, pyridyl, pyrimidinyl, triazinyl, N-oxide-pyridyl, thienyl, furyl, thiazolyl and oxazolyl, each $R_1$ being optionally substituted with an $R_2$ group;

$R_2$ is selected from the group consisting of alkyl($C_1$-$C_6$), cycloalkyl($C_3$-$C_6$), alkenyl($C_2$-$C_6$), alkynyl($C_2$-$C_6$), alkoxy ($C_1$-$C_6$), $CF_3$, CN, $SO_2$-$R_3$, $NO_2$, NH-$R_3$, $NR_3R_4$, $COR_5$, CO-$NHR_5$, $COOR_5$,

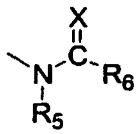 , 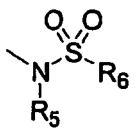 , 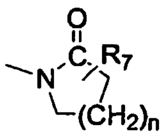 and 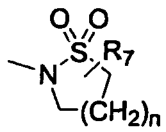 ;

$R_3$ and $R_4$ are independently selected from the group consisting of alkyl($C_1$-$C_6$), cycloalkyl($C_3$-$C_6$), aryl and heteroaryl;

$R_5$ is selected from the group consisting of hydrogen, alkyl($C_1$-$C_6$), alkenyl($C_2$-$C_6$), alkynyl($C_2$-$C_6$) and cycloalkyl ($C_3$-$C_6$);

$R_6$ is selected from the group consisting of alkyl($C_1$-$C_6$), cycloalkyl($C_3$-$C_6$), alkoxy($C_1$-$C_6$), NH-alkyl($C_1$-$C_6$), N(dialkyl ($C_1$-$C_6$)), alkyl($C_1$-$C_6$)-O-alkyl($C_1$-$C_6$), alkyl($C_1$-$C_6$)-NH-alkyl($C_1$-$C_6$), alkyl($C_1$-$C_6$)-N(dialkyl($C_1$-$C_6$)), phenyl, mono-substituted phenyl, furyl, thienyl, thiazolyl and pyridyl;

$R_7$ is selected from the group consisting of hydrogen, alkyl($C_1$-$C_6$), cycloalkyl($C_3$-$C_6$), aryl and substituted or unsubstituted heteroaryl;

$R_8$ is selected from the group consisting of hydrogen, alkyl($C_1$-$C_6$), $CF_3$, CN, CO-$R_9$ and $SO_2$-$R_9$;

$R_9$ is selected from the group consisting of hydrogen, alkyl($C_1$-$C_6$), phenyl, substituted phenyl and substituted or unsubstituted heteroaryl;

X is O, S or $NR_8$; and

n is an integer 1, 2 or 3;

and their pharmaceutically acceptable salts.

**2.** A compound according to claim 1, wherein $R_1$ is

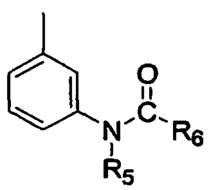

and wherein $R_5$ and $R_6$ are as described for formula (I).

**3.** A compound according to claim 2, wherein $R_5$ is selected from the group consisting of methyl, ethyl, n-propyl, i-propyl, n-butyl, cyclopropyl and 2-propynyl; and $R_6$ is selected from the group consisting of methyl, ethyl, n-propyl, n-butyl, phenyl and 4-methoxy-phenyl.

**4.** A compound according to claim 1, wherein $R_1$ is

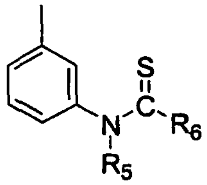

and wherein $R_5$ and $R_6$ are as described for formula (I).

**5.** A compound according to claim 4, wherein $R_5$ is selected from the group consisting of methyl, ethyl, n-propyl, i-propyl, n-butyl, cyclopropyl and 2-propynyl; and $R_6$ is selected from the group consisting of methyl, ethyl, n-propyl, n-butyl, phenyl and 4-methoxy-phenyl

**6.** A compound according to claim 1, wherein $R_1$ is

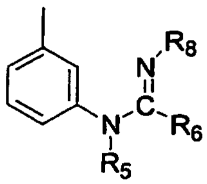

and wherein $R_5$, $R_6$ and $R_8$ are as defined for formula (I).

**7.** A compound according to claim 6, wherein $R_5$ is selected from the group consisting of methyl, ethyl, n-propyl, i-propyl, n-butyl, cyclopropyl and 2-propynyl; $R_6$ is selected from the group consisting of methyl, ethyl, n-propyl, n-butyl, phenyl and 4-methoxy-phenyl; and $R_8$ is selected from the group consisting of hydrogen, methyl and CN.

**8.** A compound according to claim 1, wherein $R_1$ is

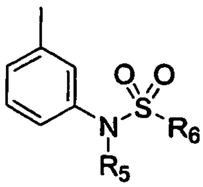

and wherein $R_5$ and $R_6$ are as defined for formula (I).

**9.** A compound according to claim 8, wherein $R_5$ is selected from the group consisting of methyl, ethyl, n-propyl, i-propyl, n-butyl, cyclopropyl and 2-propynyl; and $R_6$ is selected from the group consisting of methyl, ethyl, n-propyl, i-propyl, n-butyl, phenyl and 4-methoxy-phenyl.

**10.** A compound according to claim 1, wherein $R_1$ is

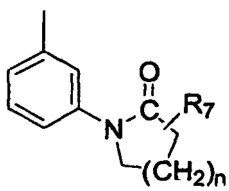

and wherein n and $R_7$ are as defined for formula (I).

**11.** A compound according to claim 10, wherein n is 1 and $R_7$ is hydrogen.

**12.** A compound according to claim 1, wherein $R_1$ is

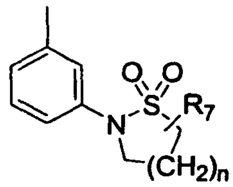

and wherein n and $R_7$ are as defined for formula (I).

**13.** A compound according to claim 12, wherein n is 1 and $R_7$ is hydrogen.

**14.** A compound according to claim 1, wherein $R_1$ is selected from the group consisting of phenyl, 2-trifluoromethylphenyl, 3-trifluoromethylphenyl, 4-trifluoromethylphenyl, furan-2-yl, thiophen-2-yl, pyridin-2-yl, pyridin-3-yl and pyridin-4-yl.

**15.** A compound according to claims 2 and 3, wherein said compound is selected from the group consisting of:

N-ethyl-N-[3-(3-nitro-pyrazolo[1,5-a]pyrimidin-7-yl)-phenyl]-acetamide;
N-methyl-N-[3-(3-nitro-pyrazolo[1,5-a]pyrimidin-7-yl)-phenyl]-acetamide;
N-[3-(3-nitro-pyrazolo[1,5-a]pyrimidin-7-yl)-phenyl]-N-(n-propyl)-acetamide;
N-(n-butyl)-N-[3-(3-nitro-pyrazolo[1,5-a]pyrimidin-7-yl)-phenyl]-acetamide; and
N-[3-(3-nitro-pyrazolo[1,5-a]pyrimidin-7-yl)-phenyl]-N-(2-propinyl)-acetamide.

**16.** A compound according to claims 8 and 9, wherein said compound is selected from the group consisting of:

N-ethyl-N-[3-(3-nitro-pyrazolo[1,5-a]pyrimidin-7-yl)-phenyl]-methanesulfonamide;
N-ethyl-N-[3-(3-nitro-pyrazolo[1,5-a]pyrimidin-7-y1)-phenyl]-4-methoxy-benzenesulfonamide;
N-ethyl-N-[3-(3-nitro-pyrazolo[1,5-a]pyrimidin-7-yl)-phenyl]-benzenesulfonamide;
N-methyl-N-[3-(3-nitro-pyrazolo[1,5-a]pyrimidin-7-yl)-phenyl]-methanesulfonamide;
N-(n-butyl)-N-[3-(3-nitro-pyrazolo[1,5-a]pyrimidin-7-yl)-phenyl]-4-methoxy-benzenesulfonamide;
N-[3-(3-nitro-pyrazolo[1,5-a]pyrimidin-7-yl)-phenyl]-N-(n-propyl)-4-methoxy-benzenesulfonamide;
N-methyl-N-[3-(3-nitro-pyrazolo[1,5-a]pyrimidin-7-yl)-phenyl]-4-methoxy-benzenesulfonamide;
N-(n-butyl)-N-[3-(3-nitro-pyrazolo[1,5-a]pyrimidin-7-yl)-phenyl]-4-benzenesulfonamide;
N-[3-(3-nitro-pyrazolo[1,5-a]pyrimidin-7-yl)-phenyl]-N-(n-propyl)-benzenesulfonamide;
N-methyl-N-[3-(3-nitro-pyrazolo[1,5-a]pyrimidin-7-yl)-phenyl]-4-benzenesulfonamide;
N-[3-(3-nitro-pyrazolo[1,5-a]pyrimidin-7-yl)-phenyl]-N-(n-propyl)-methanesulfonamide;
N-(n-butyl)-N-[3-(3-nitro-pyrazolo[1,5-a]pyrimidin-7-yl)-phenyl]-methanesulfonamide;
N-[3-(3-nitro-pyrazolo[1,5-a]pyrimidin-7-yl)-phenyl]-N-(prop-2-inyl)-methanesulfonamide;
N-[3-(3-nitro-pyrazolo[1,5-a]pyrimidin-7-yl)-phenyl]-N-(n-propyl)-ethanesulfonamide;
N-[3-(3-nitro-pyrazolo[1,5-a]pyrimidin-7-yl)-phenyl]-N-(n-ethyl)-ethanesulfonamide;
N-[3-(3-nitro-pyrazolo[1,5-a]pyrimidin-7-yl)-phenyl]-N-(n-prop-2-inyl)-propane-2-sulfonamide;
N-[3-(3-nitro-pyrazolo[1,5-a]pyrimidin-7-yl)-phenyl]-N-methyl-ethanesulfonamide;
N-[3-(3-nitro-pyrazolo[1,5-a]pyrimidin-7-yl)-phenyl]-N-(n-butyl)-ethanesulfonamide;
N-[3-(3-nitro-pyrazolo[1,5-a]pyrimidin-7-yl)-phenyl]-N-methyl-propane-2-sulfonamide;
N-[3-(3-nitro-pyrazolo[1,5-a]pyrimidin-7-yl)-phenyl]-N-ethyl-propane-2-sulfonamide;
N-[3-(3-nitro-pyrazolo[1,5-a]pyrimidin-7-yl)-phenyl]-N-(n-butyl)-propane-2-sulfonamide; and
N-[3-(3-nitro-pyrazolo[1,5-a]pyrimidin-7-yl)-phenyl]-N-(n-propyl)-propane-2-sulfonamide.

**17.** A compound according to claims 10 and 11, wherein said compound is 1-[3-(3-nitro-pyrazolo[1,5-a]pyrimidin-7-yl)-phenyl]-pyrrolidin-2-one.

**18.** A compound according to claims 12 and 13, wherein said compound is 7-(3-(2-isothiazolydinyl-1,1-dioxide)-phenyl)-3-nitro-pyrazolo[1,5-a]pyrimidine.

**19.** A compound according to claim 14, wherein said compound is selected from the group consisting of:

3-nitro-7-phenyl-pyrazolo[1,5-a]pyrimidine;
3-nitro-7-(2-trifluoromethyl-phenyl)-pyrazolo[1,5-a]pyrimidine;
3-nitro-7-(3-trifluoromethyl-phenyl)-pyrazolo[1,5-a]pyrimidine;
3-nitro-7-(4-trifluoromethyl-phenyl)-pyrazolo[1,5-a]pyrimidine;
7-furan-2-yl-3-nitro-pyrazolo[1,5-a]pyrimidine;
3-nitro-7-thiophen-2-yl-pyrazolo[1,5-a]pyrimidine;
3-nitro-7-pyridin-2-yl-pyrazolo[1,5-a]pyrimidine;
3-nitro-7-pyridin-3-yl-pyrazolo[1,5-a]pyrimidine; and
3-nitro-7-pyridin-4-yl-pyrazolo[1,5-a]pyrimidine;

**20.** A process for preparing a compound of formula (I) or a pharmaceutically acceptable salt thereof, according to claim 1, comprising reacting intermediate (II) :

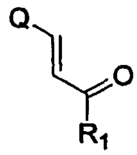

(II)

wherein $R_1$ is as defined for (I) and Q is an appropriate leaving group selected from the group consisting of N(dialkyl $(C_1-C_6)$), alkylthio$(C_1-C_6)$ and alkoxy$(C_1-C_6)$, with 4-nitro-2H-pyrazol-3-ylamine (III):

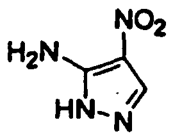

(III)

and alternatively, treatment of the compounds of claim 1, in the form of free base, with an acid to form a salt thereof.

**21.** A process according to claim 20, comprising utilizing the intermediate of formula (II) where Q is selected from the group consisting of dimethylamino, methylthio and methoxy.

**22.** A composition comprising a compound of claim 1 in association with a therapeutically inert carrier.

**23.** The use of a compound of claim 1 for preparing a medicament for treating or preventing diseases associated with $GABA_A$ receptor modulation.

**24.** The use of claim 23 wherein the diseases are associated with $\alpha_1$-$GABA_A$ or $\alpha_2$-$GABA_A$ receptor modulation.

**25.** The use of a compound of claim 1 for preparing a medicament for treating or preventing anxiety, epilepsy, sleep disorders, insomnia, for inducing sedation-hypnosis, anesthesia or muscle relaxation or for modulating the necessary time to include sleep and its duration.

## Patentansprüche

1. Verbindung der Formel 1:

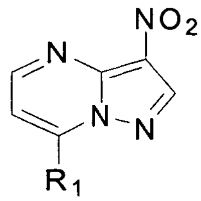

I

wobei

$R_1$ ausgewählt ist aus der Gruppe, bestehend aus Phenyl, Pyridyl, Pyrimidinyl, Triazinyl, N-Oxid-pyridyl, Thienyl, Furyl, Thiazolyl und Oxazolyl, wobei jedes $R_1$ optional mit einer $R_2$-Gruppe substituiert ist ;

$R_2$ ausgewählt ist aus der Gruppe, bestehend aus ($C_1$-$C_6$)-Alkyl, ($C_3$-$C_6$)-Cycloalkyl, ($C_2$-$C_6$)-Alkenyl, ($C_2$-$C_6$)-Alkinyl, ($C_1$-$C_6$)-Alkoxy, $CF_3$, CN, $SO_2$-$R_3$, $NO_2$, NH-$R_3$, $NR_3R_4$, $COR_5$, CO-$NHR_5$, $COOR_5$,

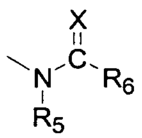 , 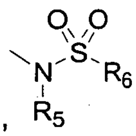 , 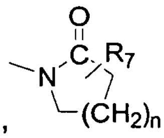 und 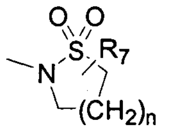 ;

$R_3$ und $R_4$ unabhängig voneinander ausgewählt sind aus der Gruppe, bestehend aus ($C_1$-$C_6$)-Alkyl, ($C_3$-$C_6$)-Cycloalkyl, Aryl und Heteroaryl;

$R_5$ ausgewählt ist aus der Gruppe, bestehend aus Wasserstoff, ($C_1$-$C_6$)-Alkyl, ($C_2$-$C_6$)-Alkenyl, ($C_2$-$C_6$)-Alkinylund ($C_3$-$C_6$)-Cycloalkyl;

$R_6$ ausgewählt ist aus der Gruppe, bestehend aus ($C_1$-$C_6$)-Alkyl, ($C_3$-$C_6$)-Cycloalkyl, ($C_1$-$C_6$)-Alkoxy, NH-($C_1$-$C_6$)-Alkyl, N(Di-($C_1$-$C_6$)-alkyl), ($C_1$-$C_6$)-Alkyl-O-($C_1$-$C_6$)-alkyl, ($C_1$-$C_6$)-Alkyl-NH-($C_1$-$C_6$)-alkyl, ($C_1$-$C_6$)-Alkyl-N (di-($C_1$-$C_6$)-alkyl), Phenyl, monosubstituiertes Phenyl, Furyl, Thienyl, Thiazolyl und Pyridyl;

$R_7$ ausgewählt ist aus der Gruppe, bestehend aus Wasserstoff, ($C_1$-$C_6$)-Alkyl, ($C_3$-$C_6$)-Cycloalkyl, Aryl und substituiertem oder unsubstituiertem Heteroaryl;

$R_8$ ausgewählt ist aus der Gruppe, bestehend aus Wasserstoff, ($C_1$-$C_6$)-Alkyl, $CF_3$, CN, CO-$R_9$ und $SO_2$-$R_9$;

$R_9$ ausgewählt ist aus der Gruppe, bestehend aus Wasserstoff, ($C_1$-$C_6$)-Alkyl, Phenyl, substituiertem Phenyl und substituiertem oder unsubstituiertem Heteroaryl;

X für O, S oder $NR_8$ steht; und

n ganzzahlig 1, 2 oder 3 ist;

und deren pharmazeutisch akzeptablen Salze.

2. Verbindung nach Anspruch 1, wobei $R_1$

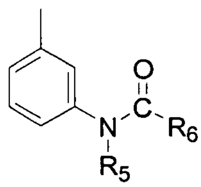

ist, und wobei $R_5$ und $R_6$ die für Formel I angegebenen Bedeutungen besitzen;

3. Verbindung nach Anspruch 2, wobei $R_5$ ausgewählt ist aus der Gruppe, bestehend aus Methyl, Ethyl, n-Propyl, i-Propyl, n-Butyl, Cyclopropyl und 2-Propinyl; und $R_6$ ausgewählt ist aus der Gruppe, bestehend aus Methyl, Ethyl,

n-Propyl, i-Propyl, n-Butyl, Phenyl und 4-Methoxy-phenyl.

**4.** Verbindung nach Anspruch 1, wobei $R_1$

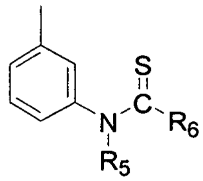

ist, und wobei $R_5$ und $R_6$ die für Formel I angegebenen Bedeutungen besitzen;

**5.** Verbindung nach Anspruch 4, wobei $R_5$ ausgewählt ist aus der Gruppe, bestehend aus Methyl, Ethyl, n-Propyl, i-Propyl, n-Butyl, Cyclopropyl und 2-Propinyl; und $R_6$ ausgewählt ist aus der Gruppe, bestehend aus Methyl, Ethyl, n-Propyl, n-Butyl, Phenyl und 4-Methoxy-phenyl.

**6.** Verbindung nach Anspruch 1, wobei $R_1$

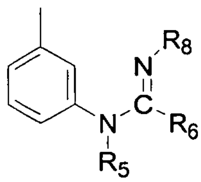

ist, und wobei $R_5$ und $R_6$ die Eigenschaften haben, wie für Formel definiert;

**7.** Verbindung nach Anspruch 6, wobei $R_5$ ausgewählt ist aus der Gruppe, bestehend aus Methyl, Ethyl, n-Propyl, i-Propyl, n-Butyl, Cyclopropyl und 2-Propinyl; $R_6$ ausgewählt ist aus der Gruppe, bestehend aus Methyl, Ethyl, n-Propyl, n-Butyl, Phenyl und 4-Methoxy-phenyl; und $R_8$ ausgewählt ist aus der Gruppe, bestehend aus Wasserstoff, Methyl und CN.

**8.** Verbindung nach Anspruch 1, wobei $R_1$

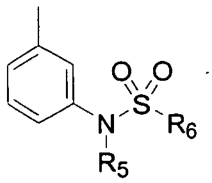

ist, und wobei $R_5$ und $R_6$ die für Formel I angegebenen Bedeutungen besitzen;

**9.** Verbindung nach Anspruch 8, wobei $R_5$ ausgewählt ist aus der Gruppe, bestehend aus Methyl, Ethyl, n-Propyl, i-Propyl, n-Butyl, Cyclopropyl und 2-Propinyl; und $R_6$ ausgewählt ist aus der Gruppe, bestehend aus Methyl, Ethyl, n-Propyl, i-Propyl, n-Butyl, Phenyl und 4-Methoxy-phenyl.

**10.** Verbindung nach Anspruch 1, wobei $R_1$

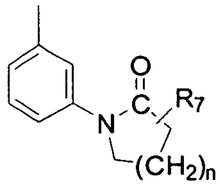

ist, und wobei n und $R_7$ die für Formel angegebenen Bedeutungen besitzen.

**11.** Verbindung nach Anspruch 10, wobei n für 1 steht und $R_7$ für Wasserstoff steht.

**12.** Verbindung nach Anspruch 1, wobei $R_1$

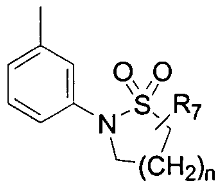

ist, und wobei n und $R_7$ die für Formel I angegebenen Bedeutungen besitzen.

**13.** Verbindung nach Anspruch 12, wobei n für 1 steht, und $R_7$ für Wasserstoff steht.

**14.** Verbindung nach Anspruch 1, wobei $R_1$ ausgewählt ist aus der Gruppe, bestehend aus Phenyl, 2-Trifluormethylphenyl, 3-Trifluormethylphenyl, 4-Trifluormethylphenyl, Furan-2-yl, Thiophen-2-yl, Pyridin-2-yl, Pyridin-3-yl und Pyridin-4-yl.

**15.** Verbindung nach den Ansprüchen 2 und 3, wobei die Verbindung ausgewählt ist aus der Gruppe, bestehend aus:

N-Ethyl-N-[3-(3-nitro-pyrazolo[1,5-a]pyrimidin-7-yl)-phenyl]-acetamid;
N-Methyl-N-[3-(3-nitro-pyrazolo[1,5-a]pyrimidin-7-yl)-phenyl]-acetamid;
N-[3-(3-Nitro-pyrazolo[1,5-a]pyrimidin-7-yl)-phenyl]-N-(n-propyl)-acetamid;
N-(n-Butyl)-N-[3-(3-nitro-pyrazolo[1,5-a]pyrimidin-7-yl)-phenyl]-acetamid; und
N-[3-(3-Nitro-pyrazolo[1,5-a]pyrimidin-7-yl)-phenyl]-N-(2-propinyl)-acetamid.

**16.** Verbindung nach den Ansprüchen 8 und 9, wobei die Verbindung ausgewählt ist aus der Gruppe, bestehend aus:

N-Ethyl-N-[3-(3-nitro-pyrazolo[1,5-a]pyrimidin-7-yl)-phenyl]-methansulfonamid;
N-Ethyl-N-[3-(3-nitro-pyrazolo[1,5-a]pyrimidin-7-yl)-phenyl]-4-methoxy-benzensulfonamid;
N-Ethyl-N-[3-(3-nitro-pyrazolo[1,5-a]pyrimidin-7-yl)-phenyl]-benzolsulfonamid;
N-Methyl-N-[3-(3-nitro-pyrazolo[1,5-a]pyrimidin-7-yl)-phenyl]-methansulfonamid;
N-(n-Butyl)-N-[3-(3-nitro-pyrazolo[1,5-a]pyrimidin-7-yl)-phenyl]-4-methoxy-benzolsulfonamid;
N-[3-(3-Nitro-pyrazolo[1,5-a]pyrimidin-7-yl)-phenyl]-N-(n-propyl)-4-methoxy-benzolsulfonamid;
N-Methyl-N-[3-(3-nitro-pyrazolo[1,5-a]pyrimidin-7-yl)-phenyl]-4-methoxy-benzolsulfonamid;
N-(n-Butyl)-N-[3-(3-nitro-pyrazolo[1,5-a]pyrimidin-7-yl)-phenyl]-4-benzolsulfonamid;
N-[3-(3-Nitro-pyrazolo[1,5-a]pyrimidin-7-yl)-phenyl]-N-(n-propyl)-benzolsulfonamid;
N-Methyl-N-[3-(3-nitro-pyrazolo[1,5-a]pyrimidin-7-yl)-phenyl]-4-benzolsulfonamid;
N-[3-(3-Nitro-pyrazolo[1,5-a]pyrimidin-7-yl)-phenyl]-N-(n-propyl)-methansulfonamid;
N-(n-Butyl)-N-[3-(3-nitro-pyrazolo[1,5-a]pyrimidin-7-yl)-phenyl]-methansulfonamid;
N-[3-(3-Nitro-pyrazolo[1,5-a]pyrimidin-7-yl)-phenyl]-N-(prop-2-inyl)-methansulfonamid;
N-[3-(3-Nitro-pyrazolo[1,5-a]pyrimidin-7-yl)-phenyl]-N-(n-propyl)-ethansulfonamid;
N-[3-(3-Nitro-pyrazolo[1,5-a]pyrimidin-7-yl)-phenyl]-N-(n-ethyl)-ethansulfonamid;
N-[3-(3-Nitro-pyrazolo[1,5-a]pyrimidin-7-yl)-phenyl]-N-(n-prop-2-inyl)-propan-2-sulfonamid;
N-[3-(3-Nitro-pyrazolo[1,5-a]pyrimidin-7-yl)-phenyl]-N-methyl-ethansulfonamid;
N-[3-(3-Nitro-pyrazolo[1,5-a]pyrimidin-7-yl)-phenyl]-N-(n-butyl)-ethansulfonamid;

N-[3-(3-Nitro-pyrazolo[1,5-a]pyrimidin-7-yl)-phenyl]-N-methyl-propan-2-sulfonamid;
N-[3-(3-Nitro-pyrazolo[1,5-a]pyrimidin-7-yl)-phenyl]-N-ethyl-propan-2-sulfonamid;
N-[3-(3-Nitro-pyrazolo[1,5-a]pyrimidin-7-yl)-phenyl]-N-(n-butyl)-propan-2-sulfonamid; und
N-[3-(3-Nitro-pyrazolo[1,5-a]pyrimidin-7-yl)-phenyl]-N-(n-propyl)-propan-2-sulfonamid.

**17.** Verbindung nach den Ansprüchen 10 und 11, wobei die Verbindung 1-[3-(3-Nitro-pyrazolo[1,5-a]pyrimidin-7-yl)-phenyl]-pyrrolidin-2-on ist.

**18.** Verbindung nach den Ansprüchen 12 und 13, wobei die Verbindung 7-(3-(2-Isothiazolidinyl-1,1-dioxid)-phenyl)-3-nitro-pyrazolo[1,5-a]pyrimidin ist.

**19.** Verbindung nach Anspruch 14, wobei die Verbindung ausgewählt ist aus der Gruppe, bestehend aus:

3-Nitro-7-phenyl-pyrazolo[1,5-a]pyrimidin;
3-Nitro-7-(2-trifluormethyl-phenyl)-pyrazolo[1,5-a]pyrimidin;
3-Nitro-7-(3-trifluormethyl-phenyl)-pyrazolo[1,5-a]pyrimidin;
3-Nitro-7-(4-trifluormethyl-phenyl)-pyrazolo[1,5-a]pyrimidin;
7-Furan-2-yl-3-nitro-pyrazolo[1,5-a]pyrimidin;
3-Nitro-7-thiophen-2-yl-pyrazolo[1,5-a]pyrimidin;
3-Nitro-7-pyridin-2-yl-pyrazolo[1,5-a]pyrimidin;
3-Nitro-7-pyridin-3-yl-pyrazolo[1,5-a]pyrimidin;
3-Nitro-7-pyridin-4-yl-pyrazolo[1,5-a]pyrimidin;

**20.** Verfahren zur Herstellung einer Verbindung der Formel 1 oder eines pharmazeutisch akzeptablen Salzes davon gemäß Anspruch 1, umfassend die Umsetzung eines Zwischenproduktes II:

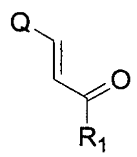

II

wobei $R_1$ wie für I definiert ist und Q eine geeignete Abgangsgruppe ist, ausgewählt aus der Gruppe, bestehend aus N(Di-($C_1$-$C_6$)-alkyl), ($C_1$-$C_6$)-Alkylthio und ($C_1$-$C_6$)-Alkoxy, mit 4-Nitro-2H-pyrazol-3-ylamin (III):

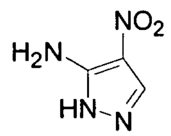

III

und alternativ die Behandlung der Verbindungen des Anspruchs 1 in Form der freien Base mit einer Säure, um ein Salz davon zu bilden.

**21.** Verfahren nach Anspruch 20, umfassend die Verwendung des Zwischenproduktes der Formel II, wobei Q ausgewählt ist aus der Gruppe, bestehend aus Dimethylamino, Methylthio und Methoxy.

**22.** Zusammensetzung, umfassend eine Verbindung nach Anspruch 1 im Zusammenhang mit einem therapeutisch inerten Träger.

**23.** Verwendung einer Verbindung nach Anspruch 1 zur Herstellung eines Medikamentes zur Behandlung oder Prävention von Krankheiten, die mit der $GABA_A$-Rezeptormodulation zusammenhängen.

**24.** Verwendung von Anspruch 23, wobei die Krankheiten mit der $\alpha_1$-$GABA_A$-Rezeptormodulation oder der $\alpha_2$-$GABA_A$-Rezeptormodulation zusammenhängen.

**25.** Verwendung einer Verbindung nach Anspruch 1 zur Herstellung eines Medikamentes zur Behandlung oder Prävention von Angst, Epilepsie, Schlafstörungen, Schlaflosigkeit, zur Einleitung von Sedations-Hypnose, Anästhesie oder Muskelrelaxation oder zur Modulierung der zum Einschlafen benötigten Zeit und der Schlafdauer.

## Revendications

**1.** Un composé de formule (I):

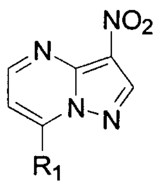

(I)

dans laquelle

$R_1$ est un groupe phényle, pyridyle, pyrimidinyle, triazinyle, N-oxyde-pyridyle, thiényle, furannyle, thiazolyle et oxazolyle, étant chaque $R_1$ éventuellement substitué par un groupe $R_2$;

$R_2$ est un groupe alcoyle($C_1$-$C_6$), cycloalcoyle($C_3$-$C_6$), alcényle($C_2$-$C_6$), alcoynyle($C_2$-$C_6$), alcoxy($C_1$-$C_6$), $CF_3$, CN, $SO_2$-$R_3$, $NO_2$, NH-$R_3$, $NR_3R_4$, $COR_5$, CO-$NHR_5$, $COOR_5$,

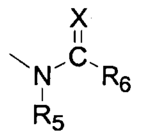 , 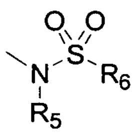 , 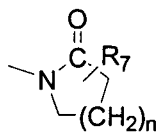 et 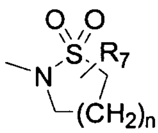 ;

$R_3$ et $R_4$ sont chacun un groupe alcoyle($C_1$-$C_6$), cycloalcoyle($C_3$-$C_6$), aryle et hétéroaryle;

$R_5$ est un groupe hydrogène, alcoyle($C_1$-$C_6$), alcényle($C_2$-$C_6$), alcoynyle($C_2$-$C_6$) et cycloalcoyle($C_3$-$C_6$);

$R_6$ est un groupe alcoyle($C_1$-$C_6$), cycloalcoyle($C_3$-$C_6$), alcoxy($C_1$-$C_6$), NH-alcoyle($C_1$-$C_6$), N(dialcoyle($C_1$-$C_6$)), alcoyle($C_1$-$C_6$)-O-alcoyle($C_1$-$C_6$), alcoyle($C_1$-$C_6$)-NH-alcoyle($C_1$-$C_6$), alcoyle($C_1$-$C_6$)-N(dialcoyle($C_1$-$C_6$)), phényle, phényle mono-substitué, furannyle, thiényle, thiazolyle et pyridyle;

$R_7$ est un groupe hydrogène, alcoyle($C_1$-$C_6$), cycloalcoyle($C_3$-$C_6$), aryle et hétéroaryle substitué ou non substitué;

$R_8$ est un groupe hydrogène, alcoyle($C_1$-$C_6$), $CF_3$, CN, CO-$R_9$ et $SO_2$-$R_9$;

$R_9$ est un groupe hydrogène, alcoyle($C_1$-$C_6$), phényle, phényle substitué et hétéroaryle substitué ou non substitué;

X est O, S ou $NR_8$; et

n est un nombre entier de 1, 2 ou 3;

et ses sels pharmaceutiquement acceptables.

**2.** Un composé selon la revendication 1, dans laquelle $R_1$ est

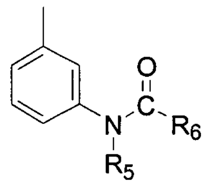

et dans laquelle $R_5$ et $R_6$ sont tels que définis pour la formule (I).

3. Un composé selon la revendication 2, dans laquelle $R_5$ est un groupe méthyle, éthyle, n-propyle, i-propyle, n-butyle, cyclopropyle et 2-propynyle; et $R_6$ est un groupe méthyle, éthyle, n-propyle, n-butyle, phényle et 4-méthoxy-phényle.

4. Un composé selon la revendication 1, dans laquelle $R_1$ est

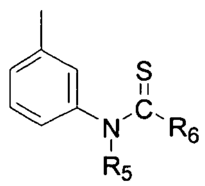

et dans laquelle $R_5$ et $R_6$ sont tels que définis pour la formule (I).

5. Un composé selon la revendication 4, dans laquelle $R_5$ est un groupe méthyle, éthyle, n-propyle, i-propyle, n-butyle, cyclopropyle et 2-propynyle; et $R_6$ est un groupe méthyle, éthyle, n-propyle, n-butyle, phényle et 4-méthoxy-phényle.

6. Un composé selon la revendication 1, dans laquelle $R_1$ est

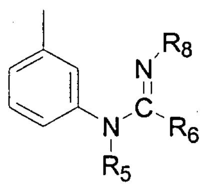

et dans laquelle $R_5$, $R_6$ et $R_8$ sont tels que définis pour la formule (I).

7. Un composé selon la revendication 6, dans laquelle $R_5$ est un groupe méthyle, éthyle, n-propyle, i-propyle, n-butyle, cyclopropyle et 2-propynyle; $R_6$ est un groupe méthyle, éthyle, n-propyle, n-butyle, phényle et 4-méthoxy-phényle; et $R_8$ est un groupe hydrogène, méthyle et CN.

8. Un composé selon la revendication 1, dans laquelle $R_1$ est

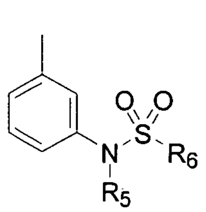

et dans laquelle $R_5$ et $R_6$ sont tels que définis pour la formule (I).

9. Un composé selon la revendication 8, dans laquelle $R_5$ est un groupe méthyle, éthyle, n-propyle, i-propyle, n-butyle, cyclopropyle et 2-propynyle; et $R_6$ est un groupe méthyle, éthyle, n-propyle, i-propyle, n-butyle, phényle et 4-méthoxy-phényle.

**10.** Un composé selon la revendication 1, dans laquelle $R_1$ est

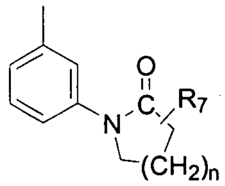

et dans laquelle n et $R_7$ sont tels que définis pour la formule (I).

**11.** Un composé selon la revendication 10, dans laquelle n est 1 et $R_7$ est hydrogène.

**12.** Un composé selon la revendication 1, dans laquelle $R_1$ est

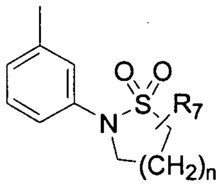

et dans laquelle n et $R_7$ sont tels que définis pour la formule (I).

**13.** Un composé selon la revendication 12, dans laquelle n est 1 et $R_7$ est hydrogène.

**14.** Un composé selon la revendication 1, dans laquelle $R_1$ est un groupe phényle, 2-trifluorométhylphényle, 3-trifluorométhylphényle, 4-trifluorométhylphényle, furan-2-yle, thiophén-2-yle, pyridin-2-yle, pyridin-3-yle et pyridin-4-yle.

**15.** Un composé selon les revendications 2 et 3, selon lesquelles ledit composé est l'un des suivants:

N-éthyl-N-[3-(3-nitro-pyrazolo[1,5-a]pyrimidin-7-yl)-phényl]-acétamide;
N-méthyl-N-[3-(3-nitro-pyrazolo[1,5-a]pyrimidin-7-yl)-phényl]-acétamide;
N-[3-(3-nitro-pyrazolo[1,5-a]pyrimidin-7-yl)-phényl]-N-(n-propyl)-acétamide;
N-(n-butyl)-N-[3-(3-nitro-pyrazolo[1,5-a]pyrimidin-7-yl)-phényl]-acétamide; et
N-[3-(3-nitro-pyrazolo[1,5-a]pyrimidin-7-yl)-phényl]-N-(2-propynyl)-acétamide.

**16.** Un composé selon les revendications 8 et 9, selon lesquelles ledit composé est l'un des suivants:

N-éthyl-N-[3-(3-nitro-pyrazolo[1,5-a]pyrimidin-7-yl)-phényl]-méthanesulfonamide;
N-éthyl-N-[3-(3-nitro-pyrazolo[1,5-a]pyrimidin-7-yl)-phényl]-4-méthoxy-benzènesulfonamide;
N-éthyl-N-[3-(3-nitro-pyrazolo[1,5-a]pyrimidin-7-yl)-phényl]-benzènesulfonamide;
N-méthyl-N-[3-(3-nitro-pyrazolo[1,5-a]pyrimidin-7-yl)-phényl]-méthanesulfonamide;
N-(n-butyl)-N-[3-(3-nitro-pyrazolo[1,5-a]pyrimidin-7-yl)-phényl]-4-méthoxy-benzènesulfonamide;
N-[3-(3-nitro-pyrazolo[1,5-a]pyrimidin-7-yl)-phényl]-N-(n-propyl)-4-méthoxy-benzènesulfonamide;
N-méthyl-N-[3-(3-nitro-pyrazolo[1,5-a]pyrimidin-7-yl)-phényl]-4-méthoxy-benzènesulfonamide;
N-(n-butyl)-N-[3-(3-nitro-pyrazolo[1,5-a]pyrimidin-7-yl)-phényl]-4-benzènesulfonamide;
N-[3-(3-nitro-pyrazolo[1,5-a]pyrimidin-7-yl)-phényl]-N-(n-propyl)-benzènesulfonamide;
N-méthyl-N-[3-(3-nitro-pyrazolo[1,5-a]pyrimidin-7-yl)-phényl]-4-benzènesulfonamide;
N-[3-(3-nitro-pyrazolo[1,5-a]pyrimidin-7-yl)-phényl]-N-(n-propyl)-méthanesulfonamide;
N-(n-butyl)-N-[3-(3-nitro-pyrazolo[1,5-a]pyrimidin-7-yl)-phényl]-méthanesulfonamide;
N-[3-(3-nitro-pyrazolo[1,5-a]pyrimidin-7-yl)-phényl]-N-(prop-2-inyl)-méthanesulfonamide;
N-[3-(3-nitro-pyrazolo[1,5-a]pyrimidin-7-yl)-phényl]-N-(n-propyl)-éthanesulfonamide;
N-[3-(3-nitro-pyrazolo[1,5-a]pyrimidin-7-yl)-phényl]-N-(n-éthyl)-éthanesulfonamide;

N-[3-(3-nitro-pyrazolo[1,5-a]pyrimidin-7-yl)-phényl]-N-(n-prop-2-inyl)-propane-2-sulfonamide;
N-[3-(3-nitro-pyrazolo[1,5-a]pyrimidin-7-yl)-phényl]-N-méthyl-éthanesulfonamide;
N-[3-(3-nitro-pyrazolo[1,5-a]pyrimidin-7-yl)-phényl]-N-(n-butyl)-éthanesulfonamide;
N-[3-(3-nitro-pyrazolo[1,5-a]pyrimidin-7-yl)-phényl]-N-méthyl-propane-2-sulfonamide;
N-[3-(3-nitro-pyrazolo[1,5-a]pyrimidin-7-yl)-phényl]-N-éthyl-propane-2-sulfonamide;
N-[3-(3-nitro-pyrazolo[1,5-a]pyrimidin-7-yl)-phényl]-N-(n-butyl)-propane-2-sulfonamide; et
N-[3-(3-nitro-pyrazolo[1,5-a]pyrimidin-7-yl)-phényl]-N-(n-propyl)-propane-2-sulfonamide.

**17.** Un composé selon les revendications 10 et 11, selon lesquelles ledit composé est:

1-[3-(3-nitro-pyrazolo[1,5-a]pyrimidin-7-yl)-phényl]-pyrrolidin-2-one.

**18.** Un composé selon les revendications 12 et 13, selon lesquelles ledit composé est:

7-(3-(2-isothiazolydinyl-1,1-dioxide)-phényl)-3-nitro-pyrazolo[1,5-a]pyrimidine.

**19.** Un composé selon la revendication 14, selon laquelle ledit composé est l'un des suivants:

3-nitro-7-phényl-pyrazolo[1,5-a]pyrimidine;
3-nitro-7-(2-trifluorométhyl-phényl)-pyrazolo[1,5-a] pyrimidine;
3-nitro-7-(3-trifluorométhyl-phényl)-pyrazolo[1,5-a] pyrimidine;
3-nitro-7-(4-trifluorométhyl-phényl)-pyrazolo[1,5-a] pyrimidine;
7-furan-2-yl-3-nitro-pyrazolo[1,5-a]pyrimidine;
3-nitro-7-thiophén-2-yl-pyrazolo[1,5-a]pyrimidine;
3-nitro-7-pyridin-2-yl-pyrazolo[1,5-a]pyrimidine;
3-nitro-7-pyridin-3-yl-pyrazolo[1,5-a]pyrimidine; et
3-nitro-7-pyridin-4-yl-pirazolo[1,5-a]pyrimidine;

**20.** Un procédé de préparation d'un composé de formule (I) et ses sels pharmaceutiquement acceptables selon la revendication 1, qui comprend que l'on fait réagir le produit intermédiaire (II):

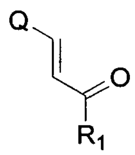

(II)

dans lequelle $R_1$ est comme défini pour (I) et Q est un groupe sortant approprié choisi dans le groupe comprenant N(dialcoyl($C_1$-$C_6$)), alcoylthio($C_1$-$C_6$) et alcoxy($C_1$-$C_6$), avec 4-nitro-2H-pyrazol-3-ylamine (III):

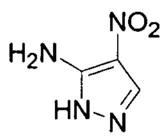

(III)

et alternativement, traitement des composés de la revendication 1, sous forme de base libre, avec un acide pour former le sel correspondant.

**21.** Un procédé selon la revendication 20, **caractérisé en ce que** l'on utilise le produit intermédiaire de formule (II) dans lequelle Q est un groupe diméthylamino, méthylthio et méthoxy.

**22.** Une composition pharmaceutique comprenant un composé selon la revendication 1 en association avec un excipient

thérapeutiquement inerte.

**23.** Utilisation d'un composé selon la revendication 1 comprenant la préparation d'un médicament pour traiter ou pour prévenir des maladies associées avec la modulation d'un récepteur $GABA_A$.

**24.** Utilisation selon la revendication 23, **caractérisée en ce que** les maladies sont associées avec la modulation de les sous-unités $\alpha_1$ ou $\alpha_2$ d'un récepteur $GABA_A$.

**25.** Utilisation d'un composé selon la revendication 1 comprenant la préparation d'un médicament pour traiter ou prévenir l'anxiété, l'épilepsie, le dysfonctionnement du sommeil, l'insomnie, pour induire la sédation-hypnose, l'anesthésie ou la relaxation musculaire ou pour moduler le temps nécessaire pour induire la sommeil et sa durée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4626538 A
- US 6399621 B
- EP 129847 A


### Non-patent literature cited in the description


- **H. MÖHLER et al.** *J. Pharmacol. Exp. Ther.,* 2002, vol. 300, 2-8
- **H. MÖHLER et al.** *Curr. Opine Pharmacol.,* 2001, vol. 1, 22-25
- **U. RUDOLPH et al.** *Nature,* 1999, vol. 401, 796-800
- **D. J. NUTT et al.** *Br. J. Psychiatry,* 2001, vol. 179, 390-396
- **C. F. P. GEORGE.** *The Lancet,* 2001, vol. 358, 1623-1626
- **J. M. DOMAGALA et al.** *J. Heterocyclic Chem.,* 1989, vol. 26 (4), 1147-58
- **K. SAWADA et al.** *Chem. Pharm. Bull.,* 2001, vol. 49 (7), 799-813
- **M. E. C. BIFFIN et al.** *J. Chem. Soc.,* 1968, 2159-2162
- **M. E. C. BIFFIN et al.** *Aust. J. Chem.,* 1967, vol. 26, 1041-1047
- **M. E. C. BIFFIN et al.** *Tetrahedron Lett.,* 1967, vol. 21, 2029-2031
- **J. LAMEH et al.** *Prog. Neuro-Psychopharmacol. Biol. Psychiatry,* 2000, vol. 24, 979-991
- **S. ARBILLA et al.** *Eur. J. Pharmacol.,* 1986, vol. 130, 257-263
- **Y. WU et al.** *Eur. J. Pharmacol.,* 1995, vol. 278, 125-132
- **Y. CHENG ; W. H. PRUSOFF.** *Biochem. Pharmacol.,* 1973, vol. 22 (23), 3099-3108
- **D. J. SANGER et al.** *Eur. J. Pharmacol.,* 1996, vol. 313, 35-42
- **G. GRIEBEL et al.** *Psychopharmacology,* 1999, vol. 146, 205-213